# EUROPEAN PATENT APPLICATION

(11) **EP 4 382 912 A1**
(43) Date of publication of application: **12.06.2024**
(21) Application number: 22849488.6
(22) Date of filing: 26.07.2022
(51) Int. Cl.: G01N 33/574, C12Q 1/6851, C12Q 1/686, G01N 33/53, G01N 33/543, G01N 33/576, G01N 33/68

(54) **DIAGNOSTIC MARKER FOR HEPATIC CANCER DEVELOPMENT IN CHRONIC HEPATIC DISEASE**

(30) Priority: 26.07.2021 JP 2021121873; 23.05.2022 JP 2022084132
(71) Applicant: OSAKA UNIVERSITY, Suita-shi Osaka 565-0871 (JP)
(72) Inventor: HIKITA, Hayato, Suita-shi, Osaka 565-0871 (JP); MYOJIN, Yuta, Suita-shi, Osaka 565-0871 (JP); TAKEHARA, Tetsuo, Suita-shi, Osaka 565-0871 (JP)
(74) Representative: J A Kemp LLP
(86) International application number: PCT/JP2022/028776
(87) International publication number: WO 2023/008427

(57) **Abstract**

The method of the present invention for evaluating the risk of developing liver cancer in a subject includes (1) a step of measuring a GDF15 level of a subject and (2) a step of relating the GDF15 level to the risk of developing liver cancer. When the GDF15 level measured in step (1) is not less than a cutoff value set in advance, the GDF15 level becomes an indicator that the subject has a high risk of developing liver cancer, and when the GDF15 level is less than the cutoff value, it becomes an indicator that the risk of developing liver cancer is low. The present invention also provides a kit for measuring GDF15 levels in a subject for use in the method of the present invention, and a diagnostic agent for evaluating the risk of developing liver cancer in a subject, which contains an anti-GDF15-specific antibody.

## Description

### [Technical Field]

The present invention relates to a method for evaluating the onset risk of diseases by using diagnostic markers for the diseases. Specifically, it relates to a method for evaluating the risk of developing liver cancer in chronic liver diseases.

### [Background Art]

Various chronic liver diseases such as chronic hepatitis B, chronic hepatitis C, nonalcoholic steatohepatitis, and the like cause liver cancer (Non Patent Literatures 1 and 2). Hepatocellular injury is observed in various chronic liver diseases, and oxidative stress is involved in liver carcinogenesis due to persistent hepatocellular injury (Non Patent Literatures 3 and 4). Chronic liver disease is classified into viral chronic liver disease and non-viral chronic liver disease. Viral chronic liver disease is classified into hepatitis C mainly due to hepatitis C virus, and hepatitis B due to hepatitis B virus. Non-viral chronic liver disease is classified into fatty liver disease and autoimmune liver disease.

### Hepatitis C

With the advent of direct acting antivirals (hereinafter referred to as "DAAs"), which are new therapeutic drugs for hepatitis C, hepatitis C virus (HCV) has been eliminated in many cases. Even after elimination of hepatitis C virus, more precisely, after sustained virological response (hereinafter referred to as "SVR"), there is a risk of liver carcinogenesis, more precisely, liver cancer development (Non Patent Literature 5). In many cases of hepatitis C, therefore, regular imaging tests and blood tests are performed after completion of the treatment. However, with the spread of DAAs treatment in recent years, the number of cases that have achieved SVR has increased, and it is problematic from a medical economic standpoint to uniformly perform regular periodic tests in all cases that have achieved SVR. Therefore, it is necessary to stratify the risk of liver carcinogenesis and to focus examination on populations with a high risk of liver carcinogenesis. In blood tests, AFP (a-fetoprotein), platelets, FIB-4 index calculated from age (also denoted as Fib-4, FIB4, or Fib4, Non Patent Literatures 6 to 8), and the like are used as tumor markers to predict cancer development, but their diagnostic ability is not sufficient and more precise stratification technique is needed.

### Hepatitis B

As of 2019, there are estimated 296 million people worldwide who are HBs antigen positive, and approximately 820,000 people die each year from liver cirrhosis and liver cancer caused by hepatitis B virus (HBV). While the risk of developing liver cancer is reduced by lowering HBV DNA in serum with a nucleic acid analog preparation (NUC), cases of cancer development are observed even in cases with low HBV DNA values (Non Patent Literature 9). The rate of liver cancer development after disappearance of HBs antigen is 0.0368/year. On the other hand, the rate of liver cancer development in HBs antigen persistently-positive cases is 0.1957/year, showing a significantly high rate of liver cancer development (Non Patent Literature 10). Examples of the factors that increase the risk of developing cancer include age (40 years or older), gender (male), high virus amount, alcohol consumption, family history of liver cancer, co-infection with HCV, HDV and/or HIV, progression of liver fibrosis, a decrease in the number of platelets reflecting the progression of liver fibrosis, genotype C, and core promoter mutations (Non Patent Literature 11). However, even when these factors are taken into account, it is difficult to predict liver cancer development under NUC administration, and new markers of liver carcinogenesis are clinically important.

### Fatty liver disease

Many of nonviral liver diseases are fatty liver diseases (so-called fatty liver). Fatty liver disease refers to a disease in which fat has accumulated in 5% or more of the liver cells. Fatty liver disease is further classified into non-alcoholic fatty liver disease (NAFLD) and secondary fatty liver. Non-alcoholic refers to alcohol consumption converted to ethanol which is less than 30 g/day for men and 20 g/day for women. NAFLD is further classified histologically into non-alcoholic fatty liver (NAFL), which is not accompanied by hepatocellular injury, and non-alcoholic steatohepatitis (NASH), which is histologically accompanied by hepatocellular injury and inflammation. Secondary fatty liver is classified into alcoholic one, drug-induced one (amiodarone, methotrexate, tamoxifen, steroid, valproic acid, antiretroviral drug, and the like), disease-related one (hepatitis C (genotype 3), Wilson disease, lipoatrophy, starvation, parenteral nutrition, Reye syndrome, acute fatty liver of pregnancy, HELLP syndrome), congenital metabolic abnormal one (abeta-lipoproteinemia, hemochromatosis, α1-antitrypsin deficiency, lecithin cholesterol acyltransferase deficiency, lysosome-producing lipase deficiency, and the like), and others (post-pancreaticoduodenectomy) .

Assuming that the population of Japan is 127 million, it is estimated that about 30% of that population (30-40 million) is affected with NAFLD, and about 10% of that population is affected with NASH. These numbers of affected people are greater than those of hepatitis C (2 million people), hepatitis B (1 million people), and alcoholic hepatitis (2 million people), and almost equal or exceed the total number of viral hepatitis cases.

In some cases of NAFL, fibrosis progresses even though slowly, whereas in some cases of NASH, fibrosis progresses, leading to liver cirrhosis or liver carcinogenesis. However, the presence of mutual migration is known between the two (Non Patent Literatures 12 and 13). As a result of a comparative study of prognosis, it has been reported that whether or not fibrosis has progressed is more important for prognosis than whether it is NAFL or NASH (Non Patent Literature 14). From another comparative study of prognosis, it has also been reported that prognosis is poor in cases with advanced liver fibrosis (Non Patent Literature 15). The study results of the cause of primary liver cancer in Japan from 1995 to 2015 have revealed that liver carcinogenesis due to nonviral liver diseases is increasing (Non Patent Literature 16).

A marker for predicting the liver cancer development from non-viral liver diseases and the usefulness of the FIB-4 index have also been reported (Gastroenterology. 2018 Dec; 155(6):1828-1837.e2). However, the diagnostic ability thereof is not sufficient and further stratification techniques are required.

Oxidative stress induces C>A/G>T gene mutations (Non Patent Literature 17), this mutation pattern is found at a higher rate in liver cancer than in other cancers (Non Patent Literature 18), suggesting the involvement of oxidative stress in liver carcinogenesis. Growth Differentiation Factor 15 (GDF15) belongs to the TGF-β superfamily and expression thereof increases in response to oxidative stress and mitochondrial stress (Non Patent Literatures 19 to 22). GDF15 elevates in advanced cases of liver fibrogenesis, and high values of GDF15 have been reported as prognostic adverse factors in liver cancer cases (Non Patent Literature 23). However, it is completely unknown whether GDF15 is related to chronic liver diseases, including the development of liver cancer after SVR in hepatitis C, the development of liver cancer under NUC administration in hepatitis B, and the development of liver cancer from NASH.

### [Citation List]

### [Non Patent Literature]

Non Patent Literature 1: Sagnelli E, et al. Infection. 2020 Feb; 48(1):7-17.
Non Patent Literature 2: Zhang CH, et al. Liver Int. 2022 doi: 10.1111/liv.15251.
Non Patent Literature 3: Hikita H, et al. J Hepatol. 2012 Jul; 57(1):92-100.
Non Patent Literature 4: Hikita H, et al. Cancer Prev Res (Phila). 2015 Aug; 8(8):693-701.
Non Patent Literature 5: Janjua NZ et al., J Hepatol 2017; 66:504-513.
Non Patent Literature 6: Watanabe T et al., J Med Virol 2020; 92:3507-3515.
Non Patent Literature 7: Kanwal F, Singal AG, Gastroenterology 2019; 157:54-64.
Non Patent Literature 8: Nagata H et al., J Hepatol 2017; 67:933-939.
Non Patent Literature 9: Liaw YF, et al. N Engl J Med. 2004; 351:1521-1531.
Non Patent Literature 10: Simonetti J, et al. Hepatology. 2010; 51:1531-1537.
Non Patent Literature 11: Yim HJ, Lok AS. HEPATOLOGY 2006; 43:S173-S181.
Non Patent Literature 12: Tokushige, K, et al. Hepatology Research.2021; 51:1013-1025.
Non Patent Literature 13: Yoshiji, H. et al. J Gastroenterol, 2021; 56:593-619.
Non Patent Literature 14: Angulo P, et al. Gastroenterology. 2015; 149:389-97.
Non Patent Literature 15: Hagstrom H, et al. J Hepatol. 2017; 67:1265-1273.
Non Patent Literature 16: Tateishi R, et al. J Gastroenterol. 2019; 54:367-376.
Non Patent Literature 17: van Loon B, et al. DNA Repair (Amst) 2010; 9:604-16
Non Patent Literature 18: Guichard C, et al. Nat Genet 2012; 44:694-8.
Non Patent Literature 19: Han ES, et al. Physiol Genomics. 2008; 34:112-26.
Non Patent Literature 20: Tsai VWW et al., Cell Metab 2018; 28:353-368.
Non Patent Literature 21: Kim J et al., Nat Metab 2021; 3:410-427.
Non Patent Literature 22: Kang SG et al., iScience 2021; 24:102181
Non Patent Literature 23: Myojin Y et al., Gastroenterology 2021; 160:1741-1754.

### [Summary of Invention]

### [Technical Problem]

The present invention aims to provide a novel biomarker that predicts liver cancer development from chronic liver diseases. The present invention further aims to provide a novel technique, which includes the novel biomarker, for the evaluation of the risk of liver carcinogenesis from and/or stratification of chronic liver diseases including, but not limited to, liver carcinogenesis after SVR in hepatitis C, liver carcinogenesis under NUC administration in hepatitis B, and liver carcinogenesis from NAFLD.

### [Solution to Problem]

The present inventors have studied to achieve the above-mentioned purposes, found that cancer development from chronic liver diseases can be predicted based on the level of GDF15 protein in the serum or the level of GDF15 transcription product in hepatic tissues, and completed the present invention.

The present invention provides a method for evaluating a risk of developing liver cancer in a subject. The method of the present invention includes
(1) a step of measuring a GDF15 level of a subject,
(2) a step of relating the aforementioned GDF15 level to the risk of developing liver cancer.

In the method of the present invention, the aforementioned subject may be at least one type of subject selected from the group consisting of a subject who achieved sustained virological response (SVR) for hepatitis C virus (HCV), a subject under NUC administration for hepatitis B, and a subject with NAFLD.

In the method of the present invention for evaluating the risk of developing liver cancer in a subject, the GDF15 level of the aforementioned subject of not less than a cutoff value set in advance becomes an indicator that the aforementioned subject has a high risk of developing liver cancer, and the GDF15 level of less than the aforementioned cutoff value can be used as an indicator that the aforementioned subject has a low risk of developing liver cancer.

In the method of the present invention for evaluating the risk of developing liver cancer in a subject, the GDF15 level measured in step (1) can be the level of GDF15 protein in serum or plasma, and/or the level of GDF15 transcription product in liver tissue or circulating blood.

In the method of the present invention for evaluating the risk of developing liver cancer in a subject, the aforementioned cutoff value may be set based on statistical analysis or ROC analysis of the aforementioned GDF15 level.

In the method of the present invention for evaluating the risk of developing liver cancer in a subject, the GDF15 level measured in step (1) may be the level of GDF15 protein in serum.

In the method of the present invention for evaluating the risk of developing liver cancer in a subject, the aforementioned cutoff value may be the median value of the level of GDF15 protein of subjects for each individual chronic liver disease, and may be determined from the ROC curve of the individual chronic liver disease.

In the method of the present invention for evaluating the risk of developing liver cancer in a subject, the cutoff value for the level of GDF15 protein in the aforementioned serum can be about 1400 pg/mL for hepatitis C patients who achieved SVR. The cutoff value for GDF15 can be about 845 pg/mL for hepatitis B patients under NUC administration. The cutoff value for GDF15 can be about 2000 pg/mL for NAFLD patients.

In the method of the present invention for evaluating the risk of developing liver cancer in a subject, the level of the GDF15 protein in the aforementioned serum can be determined by the ELISA method.

In the method of the present invention, in the step of determining the aforementioned risk of developing liver cancer, the cutoff values of AFP and FIB-4 indexes can be further combined for determination.

In the method of the present invention, the cutoff values for the aforementioned AFP and FIB-4 indexes may be respectively about 5 ng/mL and about 3.25 for hepatitis C subjects who achieved SVR and hepatitis B subjects under NUC administration. For subjects who have developed NAFLD, they may be about 5 ng/mL and about 2.67, respectively.

The present invention provides a kit for measuring GDF15 level in a subject for use in the method of the present invention. The kit of the present invention contains an anti-GDF15 antibody and/or a primer pair or a probe for specifically detecting GDF15 transcription products.

The present invention provides a diagnostic agent for evaluating the risk of developing liver cancer in a subject by the method of the present invention. The diagnostic agent of the present invention includes an anti-GDF15 antibody and/or a primer pair and a probe for specifically detecting GDF15 transcription products.

The present invention provides use of GDF15 as a biomarker for evaluating the risk of developing liver cancer in a subject, including (1) a step of measuring a GDF15 level of a subject, and (2) a step of relating the aforementioned GDF15 level to the risk of developing liver cancer.

In the use of GDF15 of the present invention as a biomarker for evaluating the risk of developing liver cancer in a subject, the aforementioned subject may be at least one type of subject selected from the group consisting of a subject who achieved sustained virological response (SVR) for hepatitis C virus (HCV), a subject under NUC administration for hepatitis B, and a subject with NAFLD.

The present invention provides a method for screening liver cancer in stratified subjects, including examining at higher frequencies a subject evaluated to have a high risk of developing liver cancer by the method of the present invention for evaluating the risk of developing liver cancer in a subject, in order to examine the presence or absence of liver cancer, than a subject evaluated to have a low risk of developing liver cancer. The presence or absence of the aforementioned liver cancer development may be examined based on ultrasound, contrast-enhanced CT imaging, MRI, and/or liver biopsy tissue findings. For subjects evaluated to have a high risk of developing liver cancer, the presence or absence of the aforementioned liver cancer development may be examined based on ultrasound, contrast-enhanced CT imaging, and MRI findings. For subjects evaluated to have a low risk of developing liver cancer, it may be possible to not perform the test for examining the presence or absence of liver cancer development, or it may be also possible to perform only the test for blood tumor markers at regular intervals, including the method of the present invention for evaluating the risk of developing liver cancer in a subject. As used herein, the blood tumor marker includes, but is not limited to, AFP (a-fetoprotein), AFP-L3 (LCA (lentil lectin) strongly binding fraction), PIVKA-II (protein induced by vitamin K absence or antagonist II) in addition to GDF15.

The present invention provides a method for preventing liver cancer, including administering a drug for preventing the development of liver cancer in a subject evaluated to have a high risk of developing liver cancer by the liver cancer screening method of the present invention. The present invention provides a drug for preventing the development of liver cancer in a subject evaluated to have a high risk of developing liver cancer by the liver cancer screening method of the present invention.

In the liver cancer screening method for hepatitis C of the present invention, subjects can be limited to those with a BMI value of less than 25 kg/m².

### [Advantageous Effects of Invention]

According to the present invention, the risk of developing liver cancer can be evaluated with high accuracy based on the GDF15 level of the subject. As a result of this, among the subjects, a liver cancer screening method including examining the presence or absence of liver cancer by tests at different frequencies and/or contents according to the risk of developing liver cancer can be performed in stratified subjects.

### [Brief Description of Drawings]

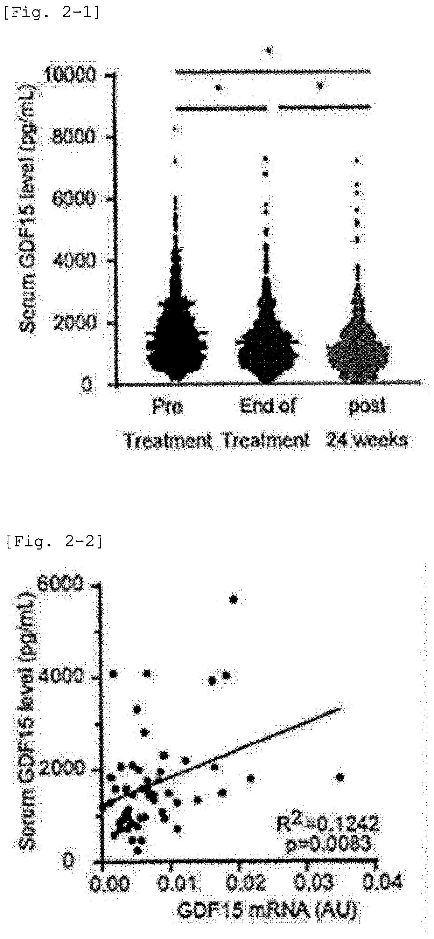

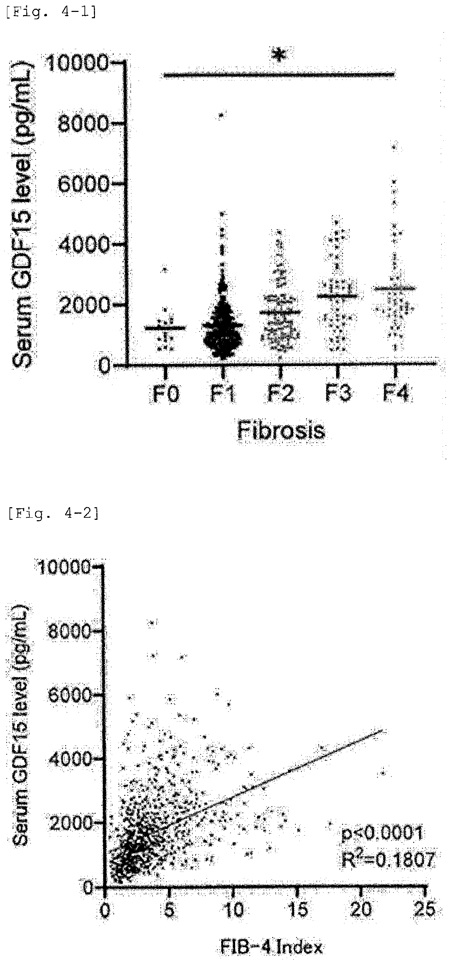

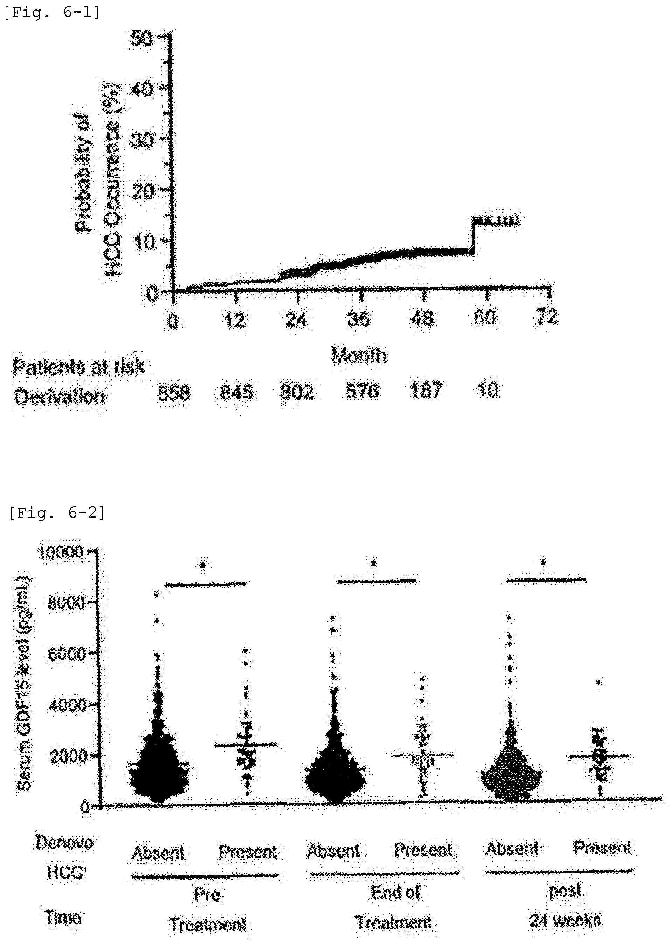

[Fig. 1]
   A table showing case details of the derivation and validation cohort of hepatitis C patients who achieved SVR.
[Fig. 2-1]
   A pyramid graph showing the distribution of GDF15 levels in stored serum collected at each time point of the derivation cohort of hepatitis C patients who achieved SVR. The vertical axis shows serum GDF15 level (pg/mL). The graph on the left shows the distribution of GDF15 levels in serum collected before treatment (Pre Treatment), the middle graph shows the distribution of GDF15 levels in serum collected at the time of completion of treatment (End of Treatment), and the graph on the right shows the distribution of GDF15 levels in serum collected 24 weeks after achieving SVR (Post 24 weeks). Asterisk (*) indicates that there is a significant difference of p<0.0001 by the Turkey Kramer test between three respective graphs in Fig. 2-1.
[Fig. 2-2]
   A graph showing the correlation between the GDF15 mRNA level in preserved liver tissue before DAA treatment of hepatitis C patients who achieved SVR and the GDF15 level in stored serum. The vertical axis shows serum GDF15 level (pg/mL), and the horizontal axis shows relative mRNA level of GDF15 (arbitrary units, AU).
[Fig. 3]
   A table showing case details of the high GDF15 group and low GDF15 group of hepatitis C patients who achieved SVR.
[Fig. 4-1]
   A pyramid graph showing the distribution of serum GDF15 level in fibrosis score groups of hepatitis C patients who achieved SVR. The vertical axis shows the serum GDF15 level (pg/mL). From the left, the distribution of serum GDF15 levels of each fibrosis score (F0 to F4) group is shown. Asterisk (*) indicates that there is a significant difference of p<0.0001 by the Test for linear trend test after one-way ANOVA between respective graphs in Fig. 4-1.
[Fig. 4-2]
   A graph showing the correlation between serum GDF15 level of hepatitis C patients who achieved SVR and FIB-4 index value. The vertical axis shows serum GDF15 level (pg/mL), and the horizontal axis shows FIB-4 index value.
[Fig. 4-3]
   A graph showing the correlation between serum GDF15 level of hepatitis C patients who achieved SVR and various parameters. The horizontal axis shows serum GDF15 level (pg/mL), and the vertical axis shows age (A), hemoglobin (B), number of platelets (C), AST(D), ALT(E), γGTP(F), eGFR(G), albumin (H), prothrombin time (I), AFP(J), and ALBI score (K).
[Fig. 5]
   A table showing case details of the derivation cohort of hepatitis C patients who achieved SVR.
[Fig. 6-1]
   A graph showing changes over time in liver cancer incidence rate in a derivation cohort of hepatitis C patients who achieved SVR. The vertical axis shows the cumulative liver cancer incidence rate, and the horizontal axis shows the observation time (months).
[Fig. 6-2]
   A pyramid graph showing the distribution of serum GDF15 level due to the presence or absence of liver cancer development in hepatitis C patients who achieved SVR, at three time points of before treatment (Pre Treatment), at the time of completion of treatment (End Of Treatment), and 24 weeks after achieving SVR (Post 24 weeks). The vertical axis shows serum GDF15 level (pg/mL). The distribution of serum GDF15 level in the groups with (Present) or without (Absent) liver cancer development is shown at three time points of before treatment (Pre Treatment), at the time of completion of treatment (End Of Treatment), and 24 weeks after achieving SVR (Post 24 weeks) from the left. Asterisk (*) indicates that there is a significant difference of p<0.005 by the Turkey Kramer test between the graphs with (Present) or without (Absent) liver cancer development at the time points of Fig. 6-2.
[Fig. 6-3]
   A graph showing changes in serum GDF15 level one year before the onset of liver cancer and at the time of onset of liver cancer for each case of liver cancer development in hepatitis C patients who achieved SVR. The vertical axis shows serum GDF15 level (pg/mL), and the horizontal axis shows observation time including one year before the onset of liver cancer (-1 year) and the time of onset of liver cancer 1(-1 year) and liver cancer development (End of Observation).
[Fig. 6-4]
   A graph showing cumulative liver cancer incidence rate in high GDF15 group and low GDF15 group of hepatitis C patients who achieved SVR. The vertical axis shows cumulative liver cancer incidence rate, and the horizontal axis shows observation time (months). The arrows "GDF15 HIGH" and "GDF15 LOW" respectively indicate graphs showing changes over time in the cumulative liver cancer incidence rate in high GDF15 group and low GDF15 group.
[Fig. 7]
   A table showing the hazard ratio after liver cancer development in the derivation cohort of hepatitis C patients who achieved SVR.
[Fig. 8]
   A graph showing ROC curves for predicting liver cancer development using GDF15 (A), AFP (B), and FIB-4 index (C) in hepatitis C patients who achieved SVR. (D) is the area under the curve (AUC) of the ROC curve. (E) shows the cutoff value, sensitivity, and specificity calculated from the ROC curve for predicting liver cancer development for GDF15, AFP, and FIB-4 index.
[Fig. 9]
   A graph showing changes over time in cumulative liver cancer incidence rate in high AFP group and low AFP group of hepatitis C patients who achieved SVR. B is a graph showing changes over time in cumulative liver cancer incidence rate in high FIB-4 index group and low FIB-4 index group of hepatitis C patients who achieved SVR. In both A and B, the vertical axis shows cumulative liver cancer incidence rate, and the horizontal axis shows observation time (months). The arrows "AFP HIGH", "AFP LOW", "FIB4-index HIGH", and "FIB4-index LOW" respectively indicate graphs showing changes over time in cumulative liver cancer incidence rate of high AFP group, low AFP group, high FIB-4 index group, and low FIB-4 index group.
[Fig. 10]
   A graph showing changes over time in cumulative liver cancer incidence rate in high risk group (3 points), middle risk group (1-2 points) and low risk group (0 point) of hepatitis C patients who achieved SVR and were stratified by a scoring system in which each high value of GDF15, AFP, or FIB-4 index is given 1 point. The vertical axis shows cumulative liver cancer incidence rate, and the horizontal axis shows observation time (months). The arrows "High", "Middle", and "Low" respectively indicate graphs showing changes over time in cumulative liver cancer incidence rate of high risk group, middle risk group, and low risk group.
[Fig. 11]
   A table showing case details of the validation cohort of hepatitis C patients who achieved SVR.
[Fig. 12]
   A graph showing changes over time in cumulative liver cancer incidence rate in a validation cohort of hepatitis C patients who achieved SVR. The vertical axis shows the cumulative liver cancer incidence rate, and the horizontal axis shows the observation time (months).
[Fig. 13]
   A graph showing changes over time in cumulative liver cancer incidence rate in high GDF15 group and low GDF15 group of hepatitis C patients who achieved SVR. B is a graph showing changes over time in cumulative liver cancer incidence rate in high AFP group and low AFP group of hepatitis C patients who achieved SVR. C is a graph showing changes over time in cumulative liver cancer incidence rate in high FIB-4 index group and low FIB-4 index group of hepatitis C patients who achieved SVR. In A, B, and C, the vertical axis shows cumulative liver cancer incidence rate, and the horizontal axis shows observation time (months). The arrows "GDF15 HIGH", "GDF15 LOW", "AFP HIGH", "AFP LOW", "FIB4-index HIGH", and "FIB4-index LOW" respectively indicate graphs showing changes over time in cumulative liver cancer incidence rate of high GDF15 group, low GDF15 group, high AFP group, low AFP group, high FIB-4 index group, and low FIB-4 index group.
[Fig. 14]
   A graph showing changes over time in cumulative liver cancer incidence rate in high risk group (3 points), middle risk group (1-2 points), and low risk group (0 point) by the scoring system of the present invention of hepatitis C patients who achieved SVR. The vertical axis shows cumulative liver cancer incidence rate, and the horizontal axis shows observation time (months). The arrows "High", "Middle", and "Low" respectively indicate graphs showing changes over time in cumulative liver cancer incidence rate of high risk group, middle risk group, and low risk group.
[Fig. 15-1]
   A graph showing changes over time in cumulative liver cancer incidence rate in high risk group (2 points), middle risk group (1 point), and low risk group (0 point) by the scoring system of the present invention, with respect to derivation cohort of hepatitis C patients who achieved SVR. The vertical axis shows cumulative liver cancer incidence rate, and the horizontal axis shows observation time (weeks). The arrows "High", "Middle", and "Low" respectively indicate graphs showing changes over time in cumulative liver cancer incidence rate of high risk group, middle risk group, and low risk group.
[Fig. 15-2]
   A graph showing changes over time in cumulative liver cancer incidence rate of the high value population and the low value population of GDF15 level in the group with low values of the known markers, AFP and FIB-4 indexes, with respect to derivation cohort of hepatitis C patients who achieved SVR. The vertical axis shows cumulative liver cancer incidence rate, and the horizontal axis shows observation time (weeks). The arrow "High" indicates a graph showing changes over time in cumulative liver cancer incidence rate of a population with low AFP and FIB-4 index values, but high GDF15 level value. The arrow "Low" indicates a graph showing changes over time in cumulative liver cancer incidence rate of a population with low AFP and FIB-4 index values, and low GDF15 level value.
[Fig. 15-3]
   A graph showing changes over time in cumulative liver cancer incidence rate in the high value population and the low value population of GDF15 level in the group with high values of the known markers, AFP and FIB-4 indexes, with respect to derivation cohort of hepatitis C patients who achieved SVR. The vertical axis shows cumulative liver cancer incidence rate, and the horizontal axis shows observation time (weeks). The arrow "High" indicates a graph showing changes over time in cumulative liver cancer incidence rate of a population with high AFP and FIB-4 index values, and high GDF15 level value. The arrow "Low" indicates a graph showing changes over time in cumulative liver cancer incidence rate of a population with high AFP and FIB-4 index values, but low GDF15 level value.
[Fig. 16]
   A scatter diagram of stored serum of hepatitis B cases under NUC administration selected according to criteria. Black circles represent cancer non-development cases, and white circles represent cancer development cases. The vertical axis shows the concentration of GDF15 (ng/mL) in the stored serum.
[Fig. 17]
   A table showing the patient backgrounds of the entire hepatitis B case cohort under NUC administration.
[Fig. 18]
   A graph showing changes over time in liver cancer incidence rate in the entire hepatitis B case cohort under NUC administration. The vertical axis shows cancer incidence rate, and the horizontal axis shows observation time (days).
[Fig. 19]
   A table showing patient backgrounds of the entire hepatitis B case cohort under NUC administration, grouped by median serum concentration of GDF15 (0.833 ng/mL).
[Fig. 20]
   A table showing the patient backgrounds of the entire hepatitis B case cohort under NUC administration, grouped according to the presence or absence of liver cancer development.
[Fig. 21]
   A graph showing the results of time-course ROC (receiver operating characteristic) curve analysis of the presence or absence of cancer development for GDF15, Fib4, AFP, and Plt in the entire hepatitis B case cohort under NUC administration at 5 years from the stored serum point. The vertical axis of each graph shows the sensitivity or true positive rate, the horizontal axis shows the false positive rate (1-specificity), and AUC shows the area under the ROC curve of each graph.
[Fig. 22]
   A graph showing the results of time-course ROC (receiver operating characteristic) curve analysis of the presence or absence of cancer development for GDF15, Fib4, AFP, and Plt in the entire hepatitis B case cohort under NUC administration at 10 years from the stored serum point. The vertical axis of each graph shows the sensitivity or true positive rate, the horizontal axis shows the false positive rate (1-specificity), and AUC shows the area under the ROC curve of each graph.
[Fig. 23]
   A graph showing changes over time in liver cancer incidence rate in the entire hepatitis B case cohort under NUC administration using the cutoff value (0.845 ng/mL) calculated from the ROC curve.
[Fig. 24]
   A table showing the results of univariate/multivariate analysis of factors contributing to cancer development using COX proportional hazards model.
[Fig. 25]
   A graph showing changes over time in liver cancer incidence rate, which was plotted by giving one point to each case with a cutoff value exceeding 5 ng/mL and 0.845 ng/mL for two types of markers of AFP and GDF15, respectively, and grouping the cases according to the scores.
[Fig. 26]
   A scatter diagram of serum concentrations of GDF15 in patients affected with NAFL or NASH. Black circles are cases without liver cancer development, and white circles are cases with liver cancer development. Five out of six cases of liver cancer development were primary hepatocellular carcinoma (HCC), and one case indicated by an arrow was cholangiocellular carcinoma (CCC).
[Fig. 27]
   A table showing backgrounds of patients affected with NAFL or NASH.
[Fig. 28]
   A table showing patient backgrounds divided into patients affected with NAFL and patients affected with NASH.
[Fig. 29]
   A graph showing changes over time in liver cancer incidence rate in the entire patient cohort affected with NAFL or NASH.
[Fig. 30]
   A scatter diagram of serum concentrations of GDF15 in cases grouped into Brunt Stage types 0 to 4 for liver fibrosis.
[Fig. 31]
   A scatter diagram examining the correlation between serum concentration of GDF15 and Fib-4 index in patients affected with NAFL or NASH.
[Fig. 32]
   A table showing hazard ratios of various attributes, blood markers, Fib-4 index, and the like of patients affected with NAFL or NASH.
[Fig. 33]
   A graph showing the results of time-course ROC (receiver operating characteristic) curve analysis of the presence or absence of cancer development for GDF15 and Fib-4 index at 5 years from the stored serum point.
[Fig. 34-1]
   A graph showing changes over time in liver cancer incidence rate with a cutoff value of 2.00 ng/mL.
[Fig. 34-2]
   A graph showing changes over time in liver cancer incidence rate with a cutoff value of 1.35 ng/mL.
[Fig. 35-1]
   Patient backgrounds of 183 cases at Ogaki Municipal Hospital.
[Fig. 35-2]
   Patient backgrounds of 170 patients in the cohort of Example 3 with an extended observation time.
[Fig. 36-1]
   A scatter diagram of stored sera of 183 patients affected with NAFL or NASH from Ogaki Municipal Hospital. Black circles are cancer non-development cases, and gray circles are cancer development cases. The vertical axis shows the concentration of GDF15 (ng/mL) in the stored serum. All nine cases of liver cancer development were primary hepatocellular carcinoma (HCC).
[Fig. 36-2]
   A scatter diagram of stored sera of 170 patients in the cohort of Example 3 with an extended observation time. Black circles are cancer non-development cases, and gray circles are cancer development cases. The vertical axis shows the concentration of GDF15 (ng/mL) in the stored serum. Seven out of eight cases of liver cancer development were primary hepatocellular carcinoma (HCC), and one case indicated by an arrow was cholangiocellular carcinoma (CCC).
[Fig. 37-1]
   Incidence rate of liver cancer in 183 cases at Ogaki Municipal Hospital.
[Fig. 37-2]
   Incidence rate of liver cancer in the cohort of Example 3 with an extended observation time.
[Fig. 38-1]
   A graph showing the results of time-course ROC (receiver operating characteristic) curve analysis of the presence or absence of cancer development at 5 years from the stored serum point for each of a total of 353 cases in the Ogaki Municipal Hospital cohort and the cohort of Example 3 with an extended observation time.
[Fig. 38-2]
   A graph showing the results of time-course ROC (receiver operating characteristic) curve analysis of the presence or absence of cancer development at 7 years from the stored serum point for each of a total of 353 cases in the Ogaki Municipal Hospital cohort and the cohort of Example 3 with an extended observation time.
[Fig. 39-1]
   A graph showing changes over time in liver cancer incidence rate with a cutoff value of 2.00 ng/mL for a total of 353 cases in the Ogaki Municipal Hospital cohort and the cohort of Example 3 with an extended observation time.
[Fig. 39-2]
   A graph showing changes over time in liver cancer incidence rate with a cutoff value of 1.74 ng/mL for a total of 353 cases in the Ogaki Municipal Hospital cohort and the cohort of Example 3 with an extended observation time.

### [Description of Embodiments]

### Definition

In the present invention, GDF15 is a cytokine belonging to the transforming growth factor (TGF) beta superfamily, and is a protein whose full-length consists of 308 amino acids. It is highly expressed in the placenta, and weakly expressed in normal tissues other than the placenta. It is rapidly up-regulated during inflammation. The amino acid sequence of human GDF15 protein and the nucleotide sequence of GDF15 mRNA have been published as NCBI Reference Sequences: NP_004855.2 and NM_004864.4, respectively, and can be isolated by methods known per se.

In the present invention, GDF15 level refers to the level of GDF15 protein and/or GDF15 transcription product. The levels of GDF15 protein and GDF15 transcription product refer to the contents of GDF15 protein and GDF15 mRNA, respectively, in a given amount of sample. In the present invention, the biological species of the GDF15 protein and GDF15 transcription product are the same as the biological species of the subject. The levels of GDF15 protein and GDF15 transcription product can be expressed in a manner known to those skilled in the art according to the below-mentioned measurement methods. For example, they may be expressed as concentrations of GDF15 protein and GDF15 transcription product, or as a relative value based on the measured value of a standard sample.

The method for measuring GDF15 protein levels uses an immunological method based on antibodies specific to GDF15 protein. GDF15 protein levels can be measured using antibody array, flow cytometry analysis, radioisotope immunoassay (RIA method), ELISA (Engvall E, Methods in Enzymol. 1980; 70:419-439.), Western blotting, immunohistostaining, enzyme immunoassay (EIA method), fluorescent immunoassay (FIA), immunochromatography method, immunoturbidimetry, immunonephelometry, and the like. ELISA is preferred from the aspects of sensitivity and ease of implementation. The details of ELISA are explained in the Example.

As the measurement methods for GDF15 transcription product level, Northern blotting method, RNase protection assay method, reverse transcription polymerase chain reaction method (RT-PCR) (Weis JH et al., Trends in Genetics 1992; 8:263-264.); quantitative real-time RT-PCR method (Held CA et al., Genome Research 1996; 6:986-994.), and the like can be used. From the aspects of sensitivity and ease of implementation, quantitative real-time RT-PCR is preferred. The details of the quantitative real-time RT-PCR method are explained in the Example.

The subject in the present invention may be any mammal, but a mammal with chronic liver disease is preferred. Examples of the mammal include experimental animals (rodents such as mouse, rat, hamster, guinea pig, and the like, and rabbit, and the like), pets such as dog, cat, and the like, domestic animals such as bovine, swine, goat, horse, sheep, and the like, primates such as monkey, orangutan and chimpanzee, and the like, human, and the like, and human is particularly preferred. The chronic liver disease here includes, but is not limited to, viral hepatitis and fatty liver disease. Viral hepatitis includes, but is not limited to, hepatitis C and hepatitis B. The fatty liver disease includes, but is not limited to, non-alcoholic fatty liver disease (NAFLD) and secondary fatty liver. NAFLD includes, but is not limited to, non-alcoholic fatty liver (NAFL) and non-alcoholic steatohepatitis (NASH). The subject in the present invention includes chronic liver disease patients whose risk of developing liver cancer needs to be evaluated. The subject in the present invention includes, but is not limited to, a subject after SVR in hepatitis C, a subject under NUC administration in hepatitis B, and a subject affected with NASH.

In the present invention, tests for examining chronic liver diseases, and the presence or absence of liver cancer development from chronic liver diseases are explained in detail in, for example, Non Patent Literatures 3 and 4.

In the present invention, sustained virological response (SVR), treatment to achieve SVR, and tests to examine the presence or absence of liver cancer development after achieving SVR respectively follow the definitions by authoritative experts of hepatitis and/or liver cancer that include, but are not limited to, Guidelines for the Management of Hepatitis C Virus Infection (edited by the Drafting Committee for Hepatitis Management Guidelines, the Japan Society of Hepatology, 8th edition, published in July, 2020 (https://www.jsh.or.jp/lib/files/medical/guidelines/j sh_guidlin es/C_v8_20201005.pdf), English version thereof (Hepatology Research 2020; 50: 791-816.)) and Clinical Practice Guidelines for Hepatocellular Carcinoma (edited by the Japan Society of Hepatology, 2017 edition, published in October, 2017 (https://www.jsh.or.jp/medical/guidelines/jsh_guidlines/medical /examination_jp_2017.html), English version thereof (https://www.jsh.or.jp/English/examination_en/guidelines_hepato cellular_carcinoma_2017.html)), Ghany MG, and Morgan TR. (Hepatitis C Guidance 2019 Update: American Association for the Study of Liver Diseases-Infectious Diseases Society of America Recommendations for Testing, Managing, and Treating Hepatitis C Viral infection. Hepatology 2020; 71:686-721.), Clinical Practice Guidelines Panel (EASL recommendations on treatment of hepatitis C: Final update of the series. J Hepatol 2020; 73:1170-1218.).

In the present invention, the treatment of hepatitis B by NUC administration follows the definitions by authoritative experts of hepatitis that include, but are not limited to, the Japan Society of Hepatology ed., Guidelines for the Management of Hepatitis B Virus Infection (version 3.4) May, 2021 (https://www.jsh.or.jp/lib/files/medical/guidelines/jsh_guidlin es/B_v3.4.pdf), English version thereof (Hepatology Research, 2020; 50: 892-923.).

In the present invention, fatty liver disease, non-alcoholic fatty liver disease (NAFLD), non-alcoholic fatty liver (NAFL), and nonalcoholic steatohepatitis (NASH)follow the definitions by authoritative experts of hepatitis that include, but are not limited to, NAFLD/NASH SINRRYO GUIDELINE 2020 (NAFLD/NASH clinical practice guideline 2020) (edited by the Japanese Society of Gastroenterology · the Japan Society of Hepatology, revised 2nd edition, published in November, 2020, Nankodo Co., Ltd. (https://www.jsge.or.jp/guideline/guideline/pdf/nafldnash2020.p df)), English version thereof (Tokushige, K. et al. HepatologyResearch.2021; 51:1013-1025. and Tokushige, K. et al. Journal of Gastroenterology 2021; 56: 951-963.).

In the present invention, the tests for examining the presence or absence of liver cancer development from viral chronic hepatitis, fatty liver disease and other liver diseases follow the definitions by authoritative experts of liver cancer that include, but are not limited to, Clinical Practice Guidelines for Hepatocellular Carcinoma (edited by the Japan Society of Hepatology, 2017 edition, the above-mentioned).

In the present invention, determining the risk of developing liver cancer includes conducting evaluations and tests based on certain criteria. Specifically, it means to determine whether or not subjects who are affected with chronic liver disease but have not developed liver cancer, including, but not limited to, subjects who have not developed liver cancer after achieving SVR for HCV, subjects who are under treatment by NUC administration for HBV and have not developed liver cancer, and subjects who are affected with other fatty liver disease such as NAFL, NASH, etc. but have not developed fatty liver cancer, are at a risk of developing liver cancer in the future, and includes determining whether or not there is a risk of developing liver cancer, and determining whether the risk of developing liver cancer is high or low. In particular, one purpose of determining the risk of developing liver cancer in the present invention is to stratify subjects according to the degree of risk of developing liver cancer, and allocate with gradient the resources for liver cancer screening tests more to subjects with a high risk of developing liver cancer than to subjects with a low risk of developing liver cancer.

In the present invention, the risk of developing liver cancer is associated with the GDF15 level of the subject. Specifically, whether the GDF15 level of a subject is higher or lower than the cutoff value determined in advance is an indicator of whether the risk of developing liver cancer is high or low for the subject.

The cutoff value in the present invention is determined by preparing a database that tracks individual GDF15 levels and the presence or absence of liver cancer development for a population of chronic liver disease patients for evaluation, and then determining the value in advance based on the statistical analysis or ROC analysis of the GDF15 level data of those who developed liver cancer and those who have not. When the aforementioned cutoff value is determined by statistical analysis, for example, the median value, arithmetical mean, or other average value of the GDF15 level data of the aforementioned population for evaluation can be used. When the aforementioned cutoff value is determined by ROC analysis, for example, the cutoff value based on the ROC analysis can be a GDF15 level on a point on the ROC curve at which the distance between the vertical axis (sensitivity or true positive rate) of the ROC curve graph is 1.0 and the horizontal axis (1-specificity) is 0.0 is minimum. Alternatively, it can be a cutoff value derived from the Youden index of the ROC curve (Cancer 1950; 3:32-35.). Once established, the database of the population of chronic liver disease patients for evaluation may be used for setting the cutoff value in the method of the present invention for evaluating the risk of developing liver cancer in a subject, without being changed at all. Alternatively, new chronic liver disease patients, including the subjects of the present invention, may be incorporated into the aforementioned population for evaluation and used for setting the cutoff value in the method for evaluating the risk of developing liver cancer in a subject according to the present invention, while appropriately updating the database of the population of chronic liver disease patients to be evaluated. As described in the Example of the present specification, the cutoff value for predicting the liver cancer development as determined from the ROC curve in the observational study by the present inventors is within the numerical range of not less than 90% and within 110% of the median value of the serum GDF15 level of the subjects in the observational study by the present inventors. Therefore, the aforementioned median value may be adopted as the aforementioned cutoff value.

In the method of the present invention for evaluating the risk of developing liver cancer in a subject, the cutoff value for the level of GDF15 protein in the aforementioned serum can be about 1400 pg/mL for hepatitis C patients who have achieved SVR. The cutoff value for GDF15 can be about 845 pg/mL for hepatitis B patients under NUC administration. The cutoff value for GDF15 can be about 2000 pg/mL for NAFLD patients. In the method of the present invention, in the step of determining the aforementioned risk of developing liver cancer, the cutoff values of AFP and FIB-4 indexes can be further combined for determination. In the method of the present invention, the cutoff values for the aforementioned AFP and FIB-4 indexes may be about 5 ng/mL and about 3.25, respectively, for hepatitis C patients who achieved SVR.

In the following, the method of the present invention for evaluating the risk of developing liver cancer in a subject is described as a method based on the GDF15 protein level of a subject and a method based on the GDF15 transcription product level of a subject.

### 1. Method for evaluating the risk of developing liver cancer in a subject affected with chronic liver disease, based on GDF15 protein level of the subject

In one embodiment of the present invention, a method for evaluating the risk of developing liver cancer in a subject affected with a chronic liver disease, including
(1) a step of measuring a GDF15 protein level of the aforementioned subject, and
(2) a step of relating the aforementioned GDF15 protein level to the risk of developing liver cancer
is provided.

The GDF15 protein level of a subject is measured using a serum or plasma sample of the subject. The plasma and serum of the subject can be prepared according to a method known per se from a peripheral blood sample collected from the subject. These may be diluted as appropriate using a known buffer and the like depending on the method used to measure the GDF15 protein level. For example, they may be diluted 75 to 100 times or more using the dilution buffer attached to the GDF15 protein human enzyme-linked immunosorbent assay (ELISA) kit (#DGD150, R&D systems, Minneapolis, MN), and the like. When it takes time from blood collection to measurement, cryopreserved plasma and/or serum can be measured.

The GDF15 protein level in step (1) can be measured by an immunological method using an antibody that specifically recognizes GDF15 protein (i.e., GDF15-specific antibody). The immunological method includes, for example, antibody array, flow cytometry analysis, radioisotope immunoassay method (RIA method), ELISA (Methods in Enzymol. 70: 419-439 (1980)), Western blotting, immunohistostaining, enzyme immunoassay (EIA method), fluorescent immunoassay (FIA), immunochromatography method, immunoturbidimetry, immunonephelometry, and the like. ELISA is preferred from the aspects of sensitivity and ease of implementation.

"Specific recognition" of antigen X by an antibody means that the affinity of the antibody for antigen X is stronger than the affinity for antigens other than antigen X in the antigen-antibody reaction. In the present specification, an antibody that specifically recognizes antigen X is sometimes abbreviated as "anti-X antibody" or "X-specific antibody".

The GDF15-specific antibody may be either a polyclonal antibody or a monoclonal antibody, or a binding fragment thereof.

The aforementioned antibody may be directly or indirectly labeled with a labeling substance. Labeling substance includes fluorescent substances (e.g., FITC, rhodamine), radioactive substances (e.g., ³²P, ³⁵S, ¹⁴C, ³H), enzymes (e.g., alkaline phosphatase, peroxidase), colored particles (e.g., metal colloidal particles, colored latex)), biotin, and the like.

Furthermore, the aforementioned antibody can be used in a soluble state with no other binding, but it may also be bound to a solid phase. Examples of the "solid phase" include plate (e.g., microwell plate), tube, bead (e.g., plastic bead, magnetic bead), chromatography carrier (e.g., water-absorbing matrix such as nitrocellulose membrane and the like, Sepharose), membrane (e.g., nitrocellulose membrane, PVDF membrane), gel (e.g., polyacrylamide gel), metal membrane (e.g., gold membrane), and the like. Among these, plate, bead, chromatography carrier, and membrane are preferably used, and plate is most preferably used in view of easy handling. Examples of the above-mentioned bond include, but are not particularly limited to, covalent bond, ionic bond, physical adsorption, and the like. Covalent bond and/or physical adsorption are preferred because they can obtain sufficient bond strength. The binding to the solid phase may be performed by direct binding to the solid phase or indirect binding to the solid phase using a substance known per se. In addition, in order to suppress nonspecific adsorption and nonspecific reactions, a phosphate buffer solution containing bovine serum albumin (BSA) or bovine milk protein is generally brought into contact with a solid phase and the surface area of the solid phase that was not coated with the antibody is blocked with the aforementioned BSA, cow milk protein, and the like.

The mode, order, specific method, and the like of the contact between the GDF15-specific antibody and the plasma or serum derived from the subject are not particularly limited as long as these antibodies can interact with GDF15 in the plasma or serum. Contact can be made, for example, by adding plasma or serum to a plate on which antibodies are immobilized. Alternatively, for example, proteins in plasma or serum may be separated by means such as SDS-PAGE, transferred to a membrane and fixed, and then brought into contact with antibodies.

Note that the time for maintaining such contact is not particularly limited as long as it is sufficient for the aforementioned antibody and GDF15 contained in the plasma or serum derived from the subject to bind and form a complex. It is generally a few seconds to several dozen hours. The temperature conditions for the contact are generally 4°C to 50°C, preferably 4°C to 37°C, and most preferably room temperature of about 15°C to 30°C. Furthermore, the pH conditions for the reaction are preferably 5.0 to 9.0, particularly preferably a neutral range of 6.0 to 8.0.

In measuring the level of GDF15 protein, the absolute value of the concentration of GDF15 protein can be easily measured in/mL units by using commercially available GDF15 protein and quantifying the ELISA results of the dilution series thereof by fluorescence, color development, or other reactions.

Then, in step (2), the level of GDF15 protein in the plasma and/or serum of the subject measured in step (1) is correlated with the risk of developing liver cancer. Correlating the level of GDF15 protein in plasma and/or serum with the risk of developing liver cancer refers to determining whether the data of the subject suggests (or indicates) the risk of developing liver cancer.

The data of the subject and the risk of developing liver cancer are generally correlated by comparing the data of the subject with the data of patients with chronic liver disease other than the subject. As shown in the Example of the present invention, it has been clarified that a group whose GDF15 protein level is higher than a cutoff value set previously (high GDF15 group) has a higher risk of developing liver cancer than a group whose GDF15 protein level is lower than the cutoff value set previously (low GDF15 group). This makes it possible to evaluate the risk of developing liver cancer in a subject.

The method for evaluating the risk of developing liver cancer of the present invention may include a step of determining the risk of developing liver cancer based on the cutoff value of GDF15 protein level. When the GDF15 protein level of the aforementioned subject is not less than the aforementioned cutoff value, the risk of developing liver cancer in the aforementioned subject is considered to be high, and when the GDF15 protein level of the aforementioned subject is less than the aforementioned cutoff value, the risk of developing liver cancer in the aforementioned subject is considered to be low. Furthermore, the aforementioned cutoff value can be the median GDF15 protein level of the population of the aforementioned subjects.

In the present specification, the adnominal adjective "about" modifying a numerical value means a numerical range of 90% or more and within 110% of the numerical value. For example, "1400 pg/mL" refers to a numerical range of not less than 1260 pg/mL and not more than 1540 pg/mL.

In the method of the present invention, the cutoff value determined as the median GDF15 protein level in the serum of hepatitis C patients who achieved SVR may be about 1400 pg/mL. This is because, in the Example of the present specification, the cutoff value for hepatitis C patients who achieved SVR, which was determined as the serum concentration of GDF15 at which the Youden index reached the maximum value according to the aforementioned ROC curve, was 1448 pg/mL, and it falls within a numerical range of not less than 90% and within 110% of the cutoff value of 1400 pg/mL determined as the aforementioned median value.

In the method of the present invention, in the aforementioned step of determining the risk of developing liver cancer, the cutoff values of AFP and/or FIB-4 index can be further combined for determination. In Hepatitis C patients who achieved SVR, it can be determined using AFP and FIB-4 indexes in combination in addition to GDF15. In Hepatitis B patients under treatment with NUC, it can be determined using AFP in combination in addition to GDF15. This is because a method of stratifying the risk of developing liver cancer in subjects who achieved SVR after DAA treatment using AFP and FIB-4 indexes as markers has been known. In the conventional techniques known to those of ordinary skill in the art, 5 ng/mL and 3.25 have been respectively used as the cutoff values for the aforementioned AFP and FIB-4 indexes.

### 2. Method for evaluating the risk of developing liver cancer in a subject affected with chronic liver disease, based on GDF15 transcription product level of the subject

In one embodiment of the present invention, a method for evaluating the risk of developing liver cancer in a subject affected with a chronic liver disease, including
(1) a step of measuring a GDF15 transcription product level of the aforementioned subject, and
(2) a step of relating the aforementioned GDF15 transcription product level to the risk of developing liver cancer is provided.

The GDF15 transcription product level of a subject is measured using a biological tissue, serum or plasma sample of the subject. The aforementioned biological tissue can be obtained, for example, by collecting a portion of the biological tissue of the subject by a method including, but not limited to, percutaneous biopsy (needle biopsy), endoscopic biopsy, or surgical biopsy. In the method of the present invention for evaluating the risk of developing liver cancer in a subject, the biological tissue is preferably hepatic tissue. Circulating GDF15 transcription product or a part thereof contained in serum or plasma samples can also be measured. In order to measure the GDF15 transcription product level of a subject, RNA can be isolated from a biological sample according to a conventional method. General methods for extracting RNA are well known in the pertinent technical field, and disclosed in molecular biology experimental protocols such as Molecular Cloning: A Laboratory Manual (3rd ed., Cold Spring Harbor Laboratory Press, 2001) by Sambrook, J and Russell, DW, and the like. Specifically, RNA can be isolated using commercially available purification kits such as RNeasy column (Qiagen, Hulsterweg, Germany) and the like and according to the manufacturer's instructions.

In order to measure the GDF15 transcription product level from isolated RNA, for example, reverse transcription polymerase chain reaction method (RT-PCR), quantitative real-time RT-qPCR method, and the like can be used. Complementary DNA is prepared from RNA by reverse transcription using GDF15-specific primers, and using the complementary DNA as a template, a reaction solution containing GDF15-specific PCR primer pair and fluorescently labeled probes is amplified using a real-time PCR device, and fluorescence can be quantified. Specifically, it can be analyzed by quantitative real-time reverse transcription polymerase chain reaction using Thunderbird qPCR Master Mix (TOYOBO, Osaka, Japan) and TaqMan probe (human GDF15, Hs00171132_m1, human beta actin Hs 9999902_m3, Applied Biosystems, Waltham, MA). In measuring the GDF15 transcription product level, the concentration of previously-synthesized GDF15 mRNA or a portion of purified RNA thereof is measured, and the dilution series thereof is amplified using a real-time PCR device and the fluorescence is quantified, whereby the absolute value of the concentration of GDF15 transcription product in RNA can be quantified. Alternatively, the relative value of the measured value of the GDF15 transcription product in RNA derived from a subject sample to be measured to the measured value of GDF15 transcription product in a control RNA at the same concentration can be quantified. In the case of relative value of the measured value of GDF15 transcription product, the unit can be arbitrary units (AU).

The primer pair and probe for specifically detecting the GDF15 transcription product used in step (1) can be synthesized based on the nucleotide sequence of human GDF15 mRNA published as NCBI Reference Sequence: NM_004864.4. The base lengths of the primers and probe are not particularly limited. The aforementioned primers can include one of a nucleotide sequence of a part of the nucleotide sequence of the aforementioned human GDF15 mRNA and a nucleotide sequence of a part of the nucleotide sequence complementary to the nucleotide sequence of the aforementioned human GDF15 mRNA as a forward primer, and the other as a reverse primer. The base length of each primer can be 10 to 50 nucleotides, preferably 15 to 30 nucleotides. The aforementioned probe includes a nucleotide sequence of a part of the nucleotide sequence complementary to the nucleotide sequence of the aforementioned human GDF15 mRNA, and the base length of the probe ranges from 10 nucleotides to the full length of the nucleotide sequence complementary to the nucleotide sequence of the aforementioned human GDF15 mRNA, preferably 20 to 150 nucleotides.

A primer pair and a probe for specifically detecting GDF15 transcription products used in step (1) may be natural nucleic acids such as RNA, DNA, and the like, as well as a combination of natural nucleic acid and chemically modified nucleic acid and pseudonucleic acid where necessary. Examples of the chemically modified nucleic acid and pseudonucleic acid include PNA (Peptide Nucleic Acid), LNA (Locked Nucleic Acid; registered trade mark), methylphosphonate-type DNA, phosphorothioate-type DNA, 2'-O-methyl-type RNA, and the like. In addition, the primers and probe may be labeled or modified using a fluorescent substance and/or a quencher substance, or a labeling substance such as radioisotope (e.g., ³²P, ³³P, ³⁵S) or the like, or biotin or (strept)avidin, or modifying substance such as magnetic beads and the like. The labeling substance is not limited and a commercially available substance can be used. For example, as a fluorescent substance, FITC, Texas, Cy3, Cy5, Cy7, Cyanine3, Cyanine5, Cyanine7, FAM, HEX, VIC, fluorescamine and derivatives thereof, and rhodamine and derivatives thereof, and the like can be used. As the quencher substance, AMRA, DABCYL, BHQ-1, BHQ-2, or BHQ-3, and the like can be used. The labeling position of the labeling substance in the primer and probe may be determined as appropriate according to the property of the modifying substance and the purpose of use. In general, it is often modified at the 5' or 3' end. Furthermore, one primer and probe molecule may be labeled with one or more kinds of labeling substances. The design of the nucleotide sequences of primer and probe and the selection of labeling substances are known and disclosed in molecular biology experimental protocols such as Molecular Cloning: A Laboratory Manual (3rd ed., Cold Spring Harbor Laboratory Press, 2001) by Sambrook, J and Russell, DW, and the like.

Then, in step (2), the level of GDF15 transcription product of the subject measured in step (1) is correlated with the risk of developing liver cancer. Correlating the level of GDF15 transcription product with the risk of developing liver cancer refers to determining whether the data of the subject suggests (or indicates) the risk of developing liver cancer.

The data of the subject and the risk of developing liver cancer are generally correlated by comparing the data of the subject with the data of patients affected with chronic liver disease other than the subject. As shown in the Example of the present invention, it has been clarified that a group whose GDF15 transcription product level is higher than a cutoff value set previously (high GDF15 group) has a higher risk of developing liver cancer than a group whose GDF15 protein level is lower than the cutoff value set previously (low GDF15 group). This makes it possible to evaluate the risk of developing liver cancer in a subject.

The method for evaluating the risk of developing liver cancer of the present invention may include a step of determining the risk of developing liver cancer based on the cutoff value of GDF15 transcription product level. When the GDF15 transcription product level of the aforementioned subject is not less than the aforementioned cutoff value, the risk of developing liver cancer in the aforementioned subject is considered to be high, and when the GDF15 transcription product level of the aforementioned subject is less than the aforementioned cutoff value, the risk of developing liver cancer in the aforementioned subject is considered to be low. Furthermore, the aforementioned cutoff value can be the median GDF15 transcription product level of the population of the aforementioned subjects.

The method for evaluating the risk of developing liver cancer of the present invention may include a step of determining the risk of developing liver cancer based on a cutoff value of the GDF15 transcription product level. When the GDF15 transcription product level of the aforementioned subject is not less than the aforementioned cutoff value, the risk of developing liver cancer of the aforementioned subject is considered to be high, and when the GDF15 transcription product level of the aforementioned subject is less than the aforementioned cutoff value, the risk of developing liver cancer of the aforementioned subject is considered to be low. Furthermore, the cutoff value can be the median GDF15 transcription product level of the population of the aforementioned subjects.

In the method of the present invention, in the step of determining the aforementioned risk of developing liver cancer, the cutoff values of AFP and FIB-4 indexes can be further combined for determination. This is because a method of stratifying the risk of developing liver cancer in subjects who achieved SVR after DAA treatment using AFP and FIB-4 indexes as markers has been known. In the conventional techniques known to those of ordinary skill in the art, 5 ng/mL and 3.25 have been respectively used as the cutoff values for the aforementioned AFP and FIB-4 indexes.

### 3. Kit for measuring GDF15 levels in a subject for use in the method of the present invention

The present invention provides a kit for measuring GDF15 levels in a subject for use in the method of the present invention. The kit of the present invention includes an anti-GDF15 antibody and/or a primer pair and a probe for specifically detecting GDF15 transcription products. The anti-GDF15-specific antibody to be contained in the kit of the present invention is as described in the present specification, "1. Method for evaluating the risk of developing liver cancer in a subject affected with chronic liver disease, based on GDF15 protein level of the subject". The primer pair and probe for specifically detecting the GDF15 transcription product contained in the kit of the present invention are as described in the section of "2. Method for evaluating the risk of developing liver cancer in a subject affected with chronic liver disease, based on GDF15 transcription product level of the subject" in the present specification.

### 4. Diagnostic agent for evaluating by the method of the present invention the risk of developing liver cancer in a subject affected with chronic liver disease

The present invention provides a diagnostic agent for evaluating the risk of developing liver cancer in a subject affected with a chronic liver disease, by the method of the present invention. The diagnostic agent of the present invention includes an anti-GDF15 antibody and/or a primer pair and a probe for specifically detecting GDF15 transcription products. The anti-GDF15-specific antibody to be contained in the diagnostic agent of the present invention is as described in the present specification, "1. Method for evaluating the risk of developing liver cancer in a subject affected with chronic liver disease, based on GDF15 protein level of the subject. The primer pair and/or probe for specifically detecting the GDF15 transcription product contained in the diagnostic agent of the present invention are as described in the section of "2. Method for evaluating the risk of developing liver cancer in a subject affected with chronic liver disease, based on GDF15 transcription product level of the subject" in the present specification.

### 5. Use of GDF15 as a biomarker for evaluating the risk of developing liver cancer in a subject

The present invention provides use of GDF15 as a biomarker for evaluating the risk of developing liver cancer in a subject. The steps of using GDF15 in the present invention are as described in the sections of "1. Method for evaluating the risk of developing liver cancer in a subject affected with chronic liver disease, based on GDF15 protein level of the subject" and "2. Method for evaluating the risk of developing liver cancer in a subject affected with chronic liver disease, based on GDF15 transcription product level of the subject" in the present specification.

All documents mentioned in the present specification are incorporated herein by reference in their entirety.

The Examples of the present invention described below are for illustrative purposes only and are not intended to limit the technical scope of the present invention. The technical scope of the present invention is limited only by the [CLAIMS]. Changes in the present invention, such as additions, deletions, and substitutions of constituent elements of the present invention, may be made without departing from the spirit of the present invention.

### [Example]

### Example 1: Evaluation of risk of developing liver cancer in subjects who achieved hepatitis C virus (HCV) sustained virological response (SVR)

### A. Material and method

### (1) Case population of research subjects

Hepatitis C cases from 26 medical institutions participating in the Osaka Liver Forum were registered at the time of baseline, and received an interferon-free DAA treatment in accordance with the guideline of the Japanese Society of Liver Studies (Hepatol Res 2020; 50:791-816.). Cases of co-infection with hepatitis B virus or human immunodeficiency virus, decompensated cirrhosis, complications of other liver diseases (autoimmune hepatitis or primary biliary cholangitis, etc.), cases after liver transplantation, and cases of under the age of 20 were excluded from the registration. By December 2017, a total of 2840 hepatitis C cases had been registered and DAA treatment was completed. Of the 2840 cases, 1609 cases at 20 facilities excluding cases where SVR was not achieved, cases for which baseline serum was not available, and cases with a history of liver cancer treatment became the subjects in the present invention. Of these, 823 cases had liver biopsy performed before DAA treatment. The histological analysis thereof was performed by Metavir score.

### (2) Clinical research review

All the members of the cases participating in the present invention submitted an informed consent document. The design of the present invention complies with the Helsinki Declaration. The patient information and sample collection protocol in the present invention were approved by the Ethical Review Board of Osaka University Hospital and the ethics committee of each institution (IRB 14148, 14419, 15080, 15325, 16314, 16494, 12449), and the analysis protocol was approved by the Institutional Review Board of Osaka University Hospital (IRB No. 17032) .

### (3) Antivirus treatment and SVR

DAA treatment was carried out by the protocols of asunaprevir and daclatasvir for 24 weeks, sofosbuvir and ledipasvir for 12 weeks, combined use of ombitasvir, paritaprevir, and ritonavir for 12 weeks, sofosbuvir and ribavirin for 12 weeks, and elbasvir and grazoprevir for 12 weeks. In the present invention, SVR means that the HCV RNA level is undetectable 24 weeks after the completion of treatment. All members of the cases were treated in accordance with the aforementioned guideline of the Japan Society of Hepatology.

### (4) Follow-up and liver cancer surveillance

All the cases before DAA treatment underwent ultrasonic test, CT and/or MRI scan to exclude cases of liver cancer development. Cases during DAA treatment underwent blood tests including hematological, biochemical, and virological tests every two weeks. Cases after treatment underwent liver cancer surveillance using ultrasound and/or CT/MRI every 6 months. In accordance with the recommendations of EASL-EORTC (European Association for the Study of the Liver - European Organisation for Research and Treatment of Cancer), and AASLD (American Association for the Study of Liver Diseases), diagnosis was performed by typical contrast-enhanced CT image and/or MRI (J Hepatol 2012; 56:908-943. and Hepatology 2018; 67:358-380.). When the image was under limitation in diagnosing liver cancer, the target biopsy of the tumor was performed and histological diagnosis was performed. The start date of follow-up was the end date of the DAA treatment. The endpoint was the day when liver cancer was developed or the date of final follow-up liver cancer surveillance imaging examination. The overall endpoint of the survival rate was the date of death from all causes or the date of the last follow-up.

### (5) Serological test

Serums from the enrolled cases were stored in a freezer at -80°C in Osaka University at the time point determined by the prospective study protocol. The GDF15 concentration in the serum was examined using a human enzyme-linked immunosorbent assay (ELISA) kit (#DGD150, R&D systems, Minneapolis, MN) according to the protocol of the manufacturer. The absorbance was examined with Varioscan LUX (Thermo Scientific, Waltham, MA).

### (6) mRNA expression analysis

Hepatic tissue RNA was extracted using an RNeasy column (Qiagen, Hulsterweg, Germany) and reversely transcribed into a complementary DNA. Messenger RNA expression was analyzed by quantitative real-time reverse transcription polymerase chain reaction using Thunderbird qPCR Master Mix (TOYOBO, Osaka, Japan) and TaqMan probe (human GDF15, Hs00171132_m1, human beta actin Hs 9999902_m3, Applied Biosystems, Waltham, MA). Target gene expression was normalized with beta-actin.

### (7) Statistical analysis

Statistical analyses for comparison of parametric and non-parametric values were respectively performed by Student t test and Mann-Whitney U test. One-way ANOVA, and subsequent Turkey-Kramer post hoc test or Kruskal-Wallis test were respectively performed for parametric and non-parametric multiple comparisons. For analysis of the liver cancer incidence rate, the DAA end date was used as an index and cases were followed until liver cancer development, death, or the final day of liver cancer surveillance before December 31, 2020, whichever came first, and the Kaplan-Meier curve was shown. A log-rank test was used to compare the liver cancer incidence rate between the two groups. Logistic regression was used to analyze liver cancer prediction, and Cox proportional hazards model was used to compare the risk of developing liver cancer. Prism ver 8.4.2 for Windows (Graph Pad PRISM RRID; SCR_014242) was used for the analysis.

### B. Results

### (1) In cases of liver cancer development after DAA treatment, serum GDF15 level was higher than in cases of no liver cancer development.

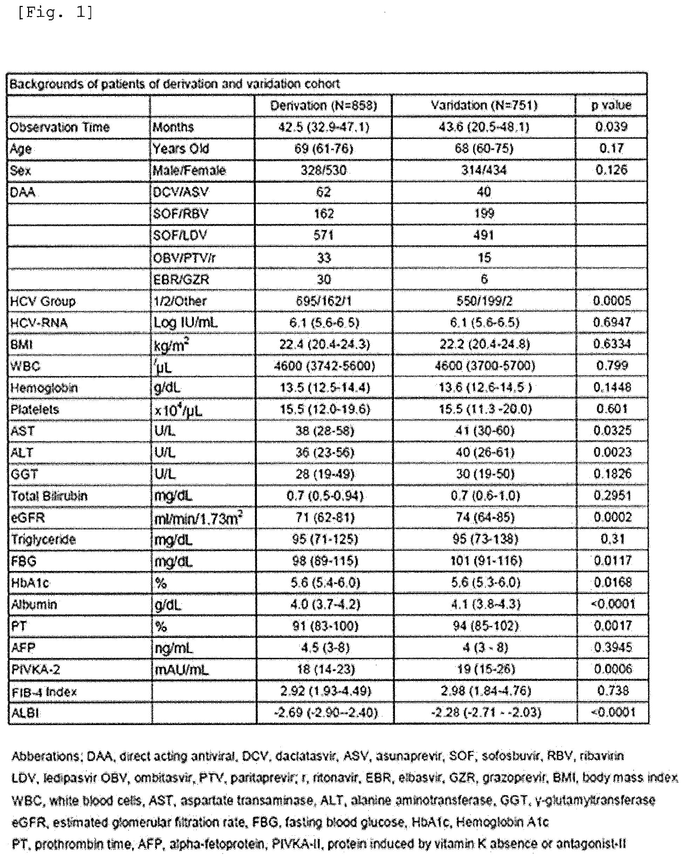

Cases without history of liver cancer treatment were divided into two groups: a derivation cohort with stored serum both at the end of treatment and 24 weeks after the end of treatment, and a validation cohort without stored serum at any time point. The details of the cases in the derivation and validation cohorts are shown in Fig. 1.

In the derivation cohort, there are stored serum at three time points: before treatment (Pre or Pre Treatment), at the end of treatment (EOT), and 24 weeks after achieving SVR (p24w or Post 24 weeks). As a result of analyzing the GDF15 level in the stored serum at the three time points, the serum GDF15 level after DAA treatment was lower than that before treatment (Fig. 2-1). In 55 cases in which liver tissues before DAA treatment were cryopreserved, a weak correlation was observed between serum GDF15 level and the expression of GDF15 in the liver (Fig. 2-2).

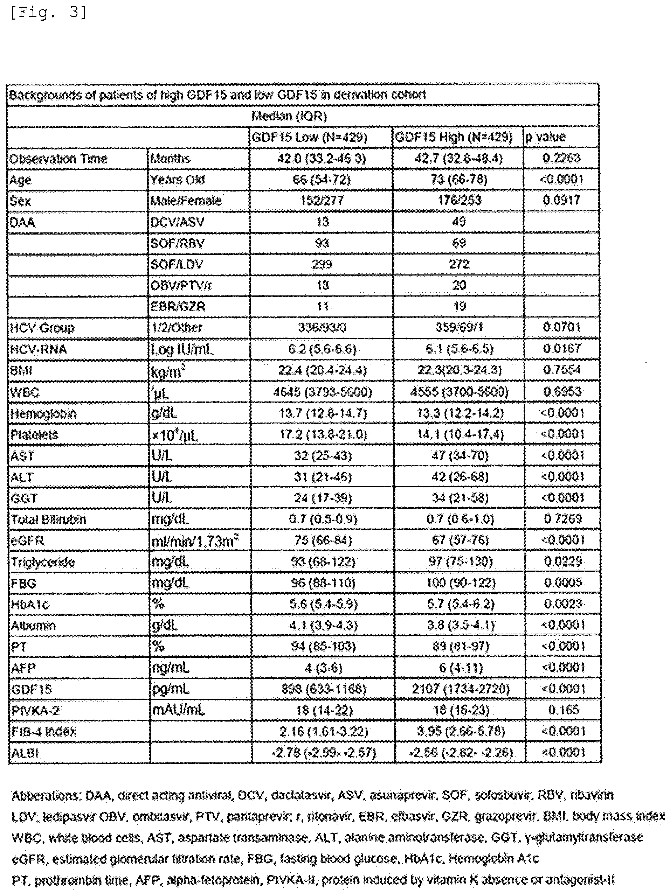

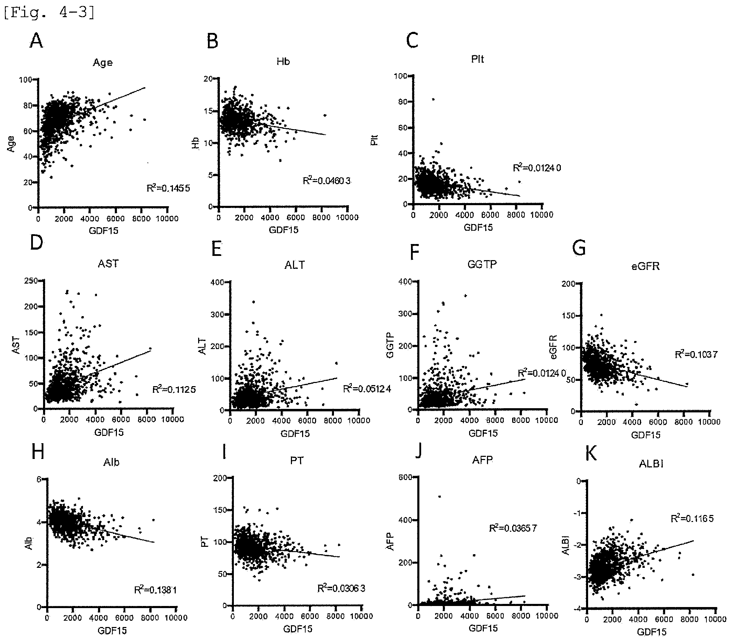

Cases were divided into two groups at 1400 pg/mL which is the median value of serum GDF15 level before treatment. As shown in the detail of the cases of the high GDF15 group and the low GDF15 group in Fig. 3, the high GDF15 group before treatment was older and had a lower number of platelet, higher AST, higher ALT, higher GGT, higher triglyceride, higher blood glucose during fasting, higher HbA1c, higher AFP, higher FIB-4 index, higher ALBI score, lower RNA level of HCV, lower hemoglobin, lower eGFR, and lower albumin, than the low GDF15 group before treatment. The serum GDF15 levels were correlated with the fibrosis scores (Fig. 4-1). The serum GDF15 levels were correlated with the FIB-4 index values (Fig. 4-2). As shown in Fig. 4-3, the serum GDF15 levels were also correlated with older age (Fig. 4-3A), high AST (Fig. 4-3D), low eGFR (Fig. 4-3G), low albumin (Fig. 4-3H), and high ALBI score (Fig. 4-3K).

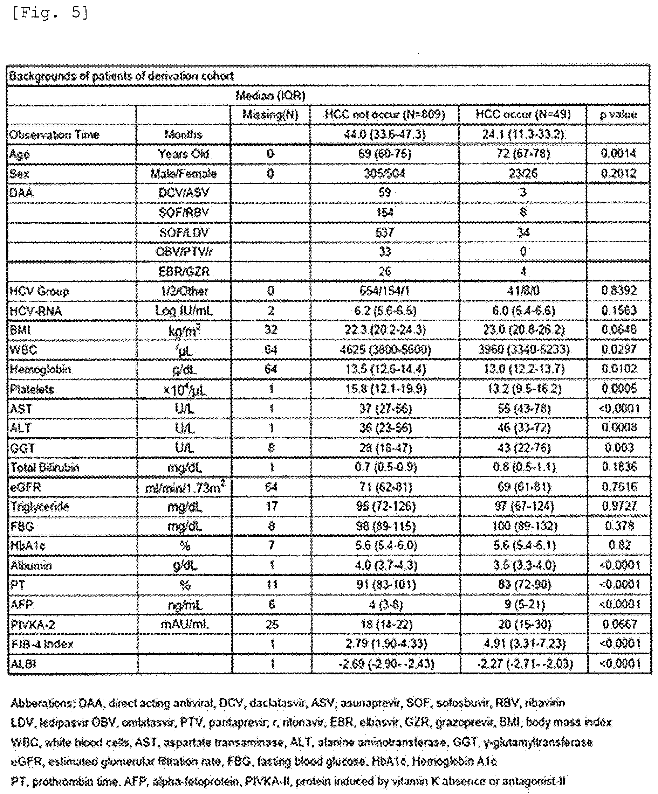

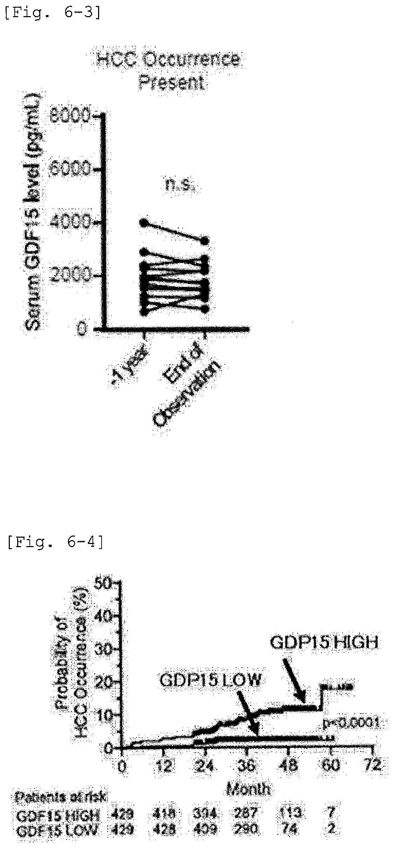

In the derivation cohort, there were 49 cases that developed liver cancer after DAA treatment within the observation time of the present invention. The details are shown in Fig 5. The liver cancer incidence rates in the derivation cohort were 1.59% for 1 year, 2.85% for 2 years, and 5.02% for 3 years (Fig. 6-1). The serum GDF15 levels of the liver cancer development cases after treatment were higher than those of the liver cancer non-development cases after treatment at any of the three time points: before treatment (Pre Treatment), at the end of treatment (End Of Treatment), and 24 weeks after achieving SVR (Post 24 weeks) (Fig. 6-2). There was no significant change in GDF15 levels at the liver cancer development compared to GDF15 levels one year before the liver cancer development (Fig. 6-3). This suggests that the serum GDF15 levels may reflect the seriousness of liver disease at that time point in each case, unlike tumor markers such as AFP and PIVKA2.

The cumulative liver cancer incidence rates over 1, 2 and 3 years were 2.80%, 4.44%, and 8.31%, respectively, in the high GDF15 group, and 0.47%, 1.12%, and 1.93%, respectively, in the low GDF15 group. The cumulative liver cancer incidence rates over 1, 2, and 3 years were significantly lower in the low GDF15 group than in the high GDF15 group (Fig. 6-4).

### (2) Serum GDF15 level may be novel biomarker that predicts development of liver cancer after DAA treatment

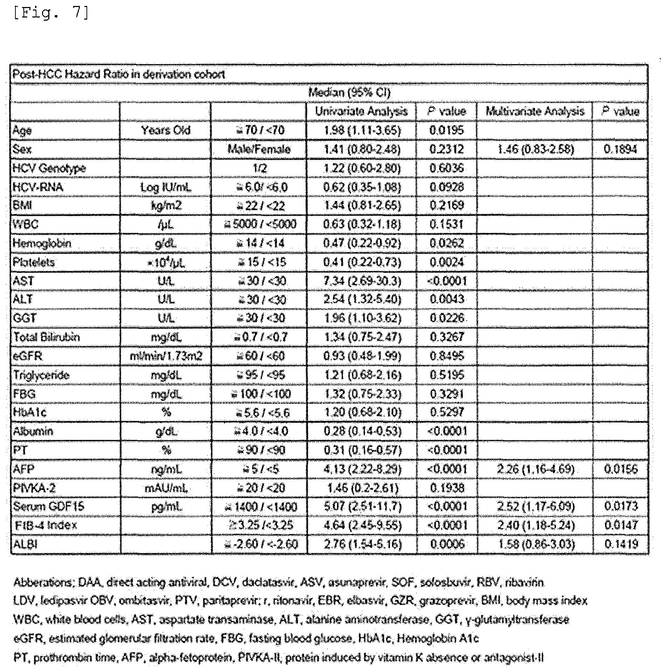

In order to examine serum GDF15 level before treatment as a biomarker predicting the development of liver cancer, variables before treatment, which are associated with the development of liver cancer, were analyzed using a Cox Hazard model. Cases with a FIB-4 index greater than 3.25 (>3.25) were considered to be liver fibrosis progressive cases based on past findings (Gastroenterology 2017; 153:996-1005.e1001., and Hepatology 2007; 46:32-36.). Other variables were assigned to two groups based on a median value or past findings (Clin Gastroenterol Hepatol 2014; 12:1186-1195.). A univariate analysis showed that older age, low platelet, high AST, high ALT, low albumin, low prothrombin activity, high AFP, high serum GDF15, high FIB-4 index, and high ALBI score were associated with an increase in the risk of developing liver cancer (Fig. 7).

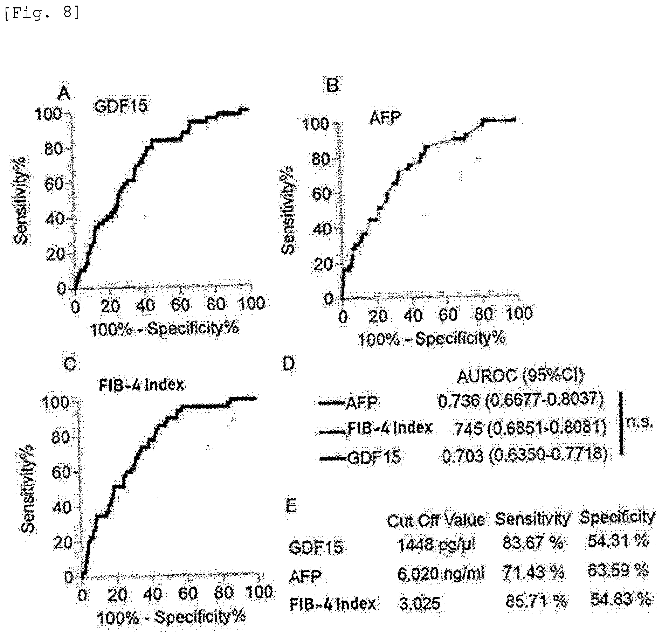

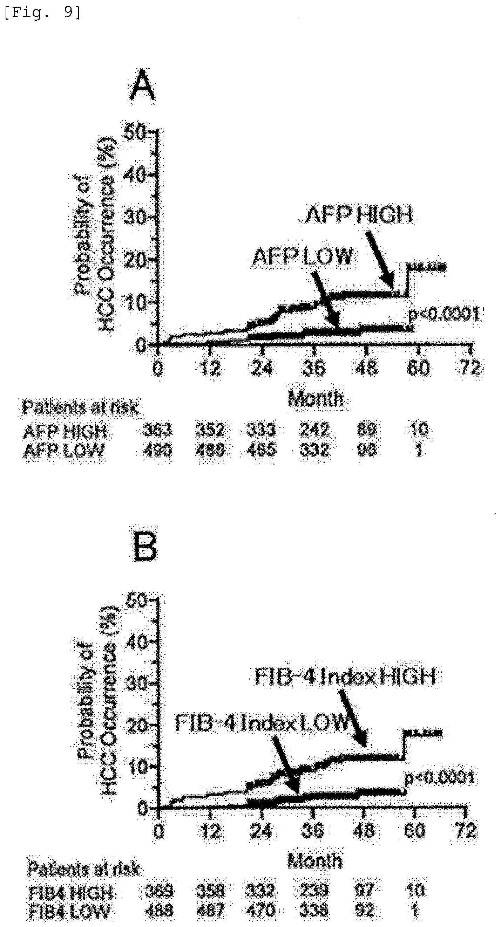

Parameters of gender, AFP as a tumor marker, GDF15 level, FIB-4 index as the progression degree of fibrosis calculated by age, AST, ALT, and platelets, and ALBI score calculated by albumin and bilirubin were selected for a multivariate Cox regression model. Among these parameters, AFP (J Med Virol 2020; 92:3507-3515. and J Hepatol 2017; 67:933-939.), FIB-4 index (Gastroenterology 2017; 153:996-1005.e1001., J Med Virol 2020; 92:3507-3515. and J Hepatol 2017; 68:25-32.), and ALBI score (Dig Liver Dis 2019; 51:681-688.) are known to be risk factors for liver cancer after HCV elimination. High GDF15 values (HR 2.52 95% CI 1.17-6.09), high AFP (HR 2.26 95% CI 1.16-4.69), and high FIB-4 indexes (HR 2.40 95%CI 1.18-5.24) were independently associated with an increase in the risk of developing liver cancer (Fig. 7). There was no significant difference in the prediction of liver cancer development among AFP, FIB-4 index, and GDF15 (Fig. 8). The cumulative liver cancer incidence rates were 8-9% over 3 years in the high AFP group or the high FIB-4 index group, and 2-3% over 3 years in the low AFP group or the low FIB-4 index group (Figs. 9A and 9B) .

The cutoff values of GDF15, AFP, and FIB-4 index for predicting liver cancer development were determined by the ROC curve at Youden index (Cancer 1950; 3:32-35.). The cutoff values of serum GDF15, AFP, and FIB-4 index were 1448 pg/mL, 6.020 ng/mL, and 3.025, respectively (Fig. 8E). These cutoff values were similar to 1400 pg/mL, 5 ng/mL, and 3.25, which were the median values of serum GDF15 levels before treatment (Fig. 7).

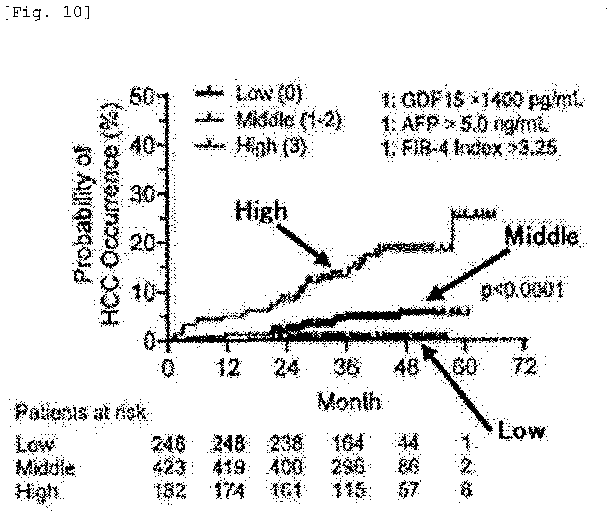

In order to stratify the risk of developing liver cancer, the total score of each case was calculated, wherein each case with a high value of GDF15, AFP, or FIB-4 index was 1 point. A score of 0 was defined as a low risk group, a score of 1 or 2 was defined as a middle risk group, and a score of 3 was defined as a high risk group. The cumulative risks of developing liver cancer over 1, 2, and 3 years were 0%, 0.40%, and 0.40% in the low risk group, 1.18%, 1.89%, and 4.44% in the middle risk group, and 4.95%, 8.26%, and 13.2% in the high risk group (Fig. 10).

Of the 248 low risk cases, only one developed liver cancer. The BMI of the case was 31.4 kg/m² and the HbA1c was 6.4%. In Japan, a population with a BMI exceeding 30 kg/m² is rare, and also in the present invention, the percentage of the cases with a BMI exceeding 30 g/m² in both the derivation and validation cohorts was 2.5%. Therefore, it is a future problem to investigate whether GDF15 is as effective in predicting the risk of developing liver cancer in cases with a BMI exceeding 30 kg/m² in the same way as in the cases with a normal BMI.

### (3) Scoring system using GDF15 level, AFP, and FIB-4 index can stratify the risk of developing liver cancer in validation cohort.

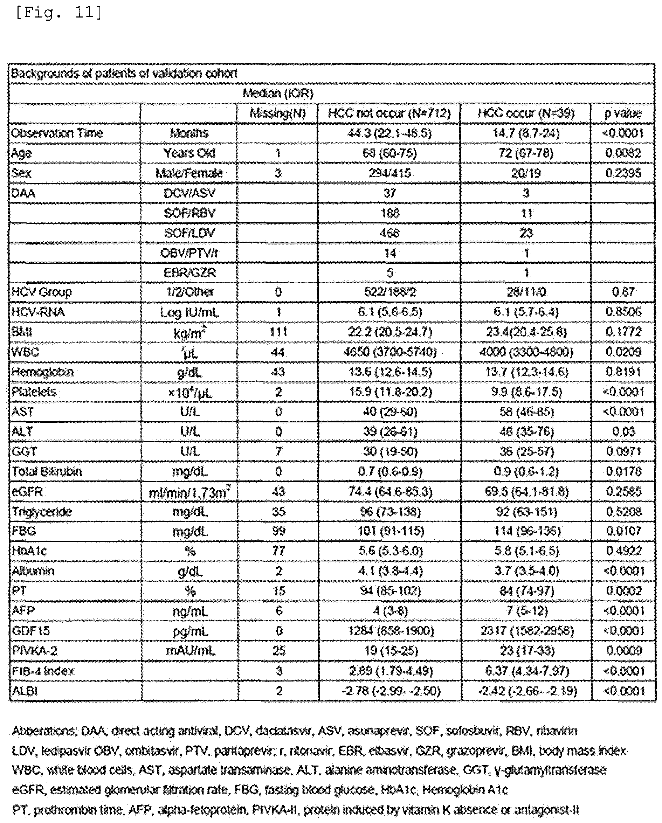

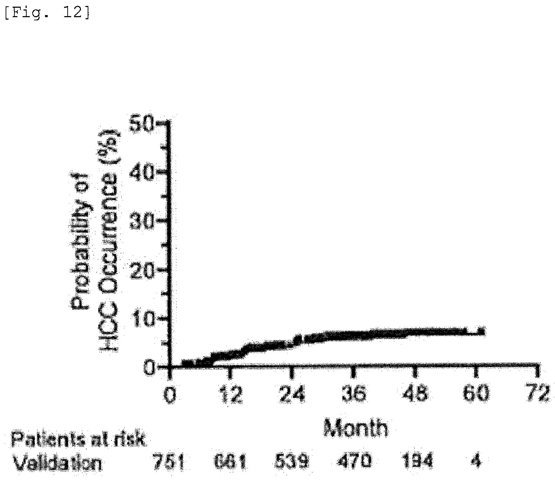

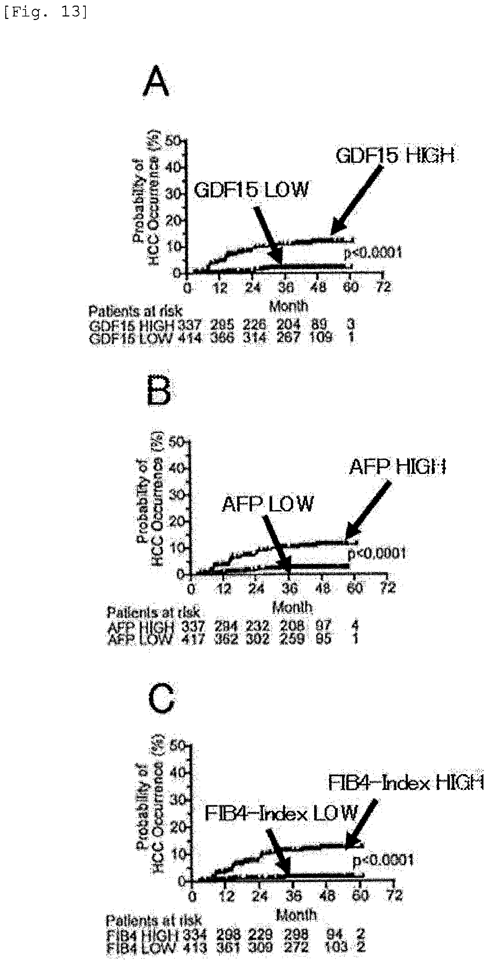

In the study of a derivation cohort, the risk of developing liver cancer was stratified by a scoring system using GDF15 level, AFP, and FIB-4 index. The scoring system was verified using a validation cohort of 751 cases for whom only serum before DAA treatment was usable. In the validation cohort, 39 cases developed liver cancer after DAA treatment during the observation time (Fig. 11). The liver cancer incidence rates in the validation cohort were 1.95% over 1 year, 4.54% over 2 years, and 5.82% over 3 years (Fig. 12). There was no significant difference in the cumulative liver cancer incidence rate between the derivation cohort and the validation cohort (p=0.57). The cumulative liver cancer incidence rates in the low GDF15 group, the low AFP group, and the low FIB-4 index group were respectively significantly lower than those in the high GDF15 group, the high AFP group, and the high FIB-4 index group (Figs. 13A-13C).

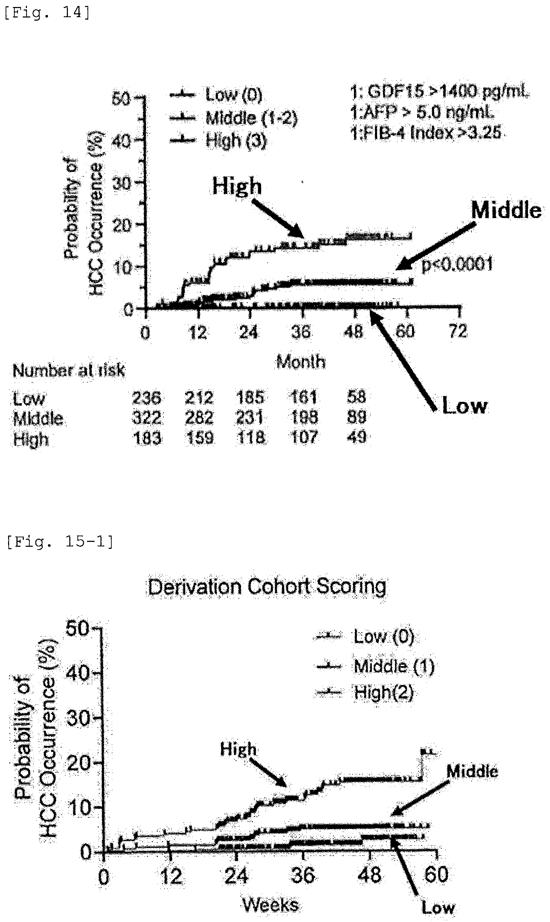

In this scoring system, a high risk population and a low risk population were narrowed down and the risk of developing liver cancer was clearly stratified (Fig. 14). The cumulative risks of developing liver cancer over 1, 2, and 3 years were 6.12%, 13.43%, and 14.2% in the high risk group (3 points, N=183), and 1.0%, 2.91%, and 5.52% in the middle risk group (1-2 points, N=322). Importantly, the low risk group (0 point, N=236) did not develop liver cancer at all (Fig. 14).

In order to further clarify the significance of the stratification of the risk of developing liver cancer after SVR by a scoring system that combines the novel biomarker GDF15 with a known marker AFP, and FIB-4 index, a comparison was made in the following with the stratification results of the risk of developing liver cancer after SVR in the derivation cohort of the present invention by a scoring system using only the known markers AFP and FIB-4 indexes.

For the derivation cohort, a total score for each case was calculated wherein a high value of each of the known markers AFP and FIB-4 indexes was 1 point. A score of 0 was defined as a low risk group, a score of 1 was defined as a middle risk group, and a score of 2 was defined as a high risk group. As shown in Fig. 15-1, the degree of stratification of the Kaplan-Meier curve for each risk group was rough, and a liver cancer incidence rate comparable to that in the middle risk group was observed even in the low risk group.

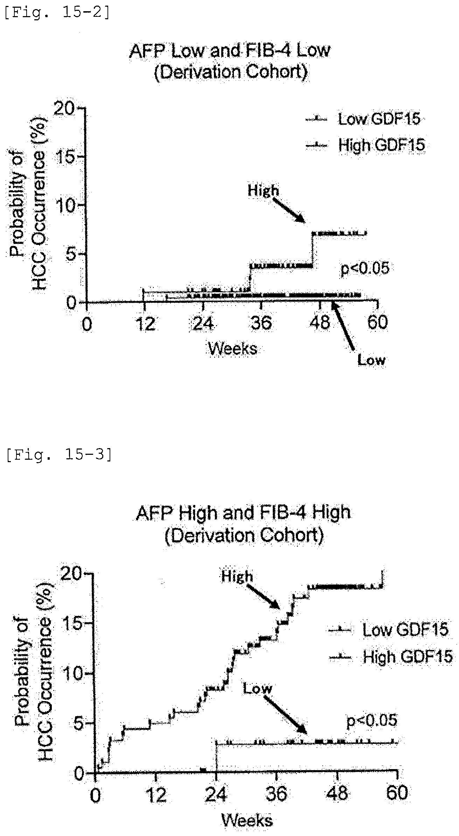

When a low risk group in a scoring system using only the known markers AFP and FIB-4 indexes was stratified into a population with low values of both AFP and FIB-4 indexes but a high value of GDF15 level, and a population with low values of both AFP and FIB-4 indexes and a low value of GDF15 level, as shown in Fig. 15-2, the liver cancer incidence rate was 0 in the population with low values of both AFP and FIB-4 indexes and low GDF15 level. Therefore, since there are no cases of liver cancer for the low GDF15, low AFP, and low FIB-4 index groups by stratifying the risk of developing liver cancer after SVR using a scoring system that uses the novel biomarker GDF15 in combination with the known markers AFP and FIB-4 indexes, the present invention has demonstrated for the first time that the risk of developing liver cancer after DAA treatment is extremely low.

In contrast, in a population with low AFP and low FIB-4 index and high GDF15 level, the liver cancer incidence rate was about 7% over 1 year. Therefore, it was confirmed that the risk of developing liver cancer after DAA treatment is not sufficiently low only by stratification with low AFP and low FIB-4 index.

When a high risk group in a scoring system using only the known markers AFP and FIB-4 indexes was stratified into a population with a high value of GDF15 level, and a population with a low value of GDF15 level, as shown in Fig. 15-3, a large difference was found in the liver cancer incidence rate after DAA treatment between a population with high values of both AFP and FIB-4 indexes but a low value of GDF15 level, and a population with high values of both AFP and FIB-4 indexes and a high value of GDF15 level. This shows that stratification by GDF15 contributes more to the prediction of the liver cancer incidence rate after DAA treatment than stratification by known markers, AFP and FIB-4 indexes. From the above analysis, the usefulness of the scoring system including the novel biomarker GDF15 was verified.

The Examples of the present invention are retrospective studies using stored serum, and a bias related to the applicability of serum cannot be denied. To eliminate this concern, the derivation cohort and the validation cohort were compared to show the absence of significant difference at least in the liver cancer incidence rate. Myojin, Y. et al. (Aliment Pharmacol Ther. 2022; 55:422-433) analyzed a derivation cohort (964 cases) and a validation cohort (642 cases) wherein cases of the derivation cohort and validation cohort of the Examples were randomly divided into 3:2. As a result, the cutoff values determined by the ROC curves for serum GDF15, AFP, and FIB-4 index at which the Youden index reached the maximum value were 1350 pg/mL, 5 ng/mL, and 3.25, respectively. These numerical values were similar to the median value of the serum GDF15 level before treatment in the present Examples.

### Example 2: Evaluation of risk of developing liver cancer in subject who is under treatment with NUC administration for hepatitis B virus (HBV) and has not developed liver cancer

### A. Material and method

### (1) Case population of research subjects

The research subject of this Example is a case of nucleic acid analog (NUC) administration with stored serum permitting investigation of progress for a long term. Here, the selection criteria for the stored serum are that the stored serum point (when multiple stored serum points exist, the oldest serum point among them) has a history of NUC administration for 8 months or more, and the stored serum point is serum HBV DNA less than 3.0 log IU/ml. Patients with a history of liver cancer at the time of the serum point and patients with combined liver diseases other than hepatitis B are excluded.

### (2) Clinical research review

The design of the present invention complies with the Helsinki Declaration. The patient information and sample collection, analysis protocol in the present invention were approved by the Ethical Review Board of Osaka University Hospital (IRB 17032), and permitted by each facility including Osaka University Hospital.

### (3) Antivirus treatment

Treatment was performed in accordance with the guidelines available at that time from "Guidelines for the Management of Hepatitis B Virus Infection" edited by the Japan Society of Hepatology (the first version) April, 2013 - (version 3.4) May, 2021 (https://www.jsh.or.jp/lib/files/medical/guidelines/j sh_guidlin es/B_v3.4.pdf), English version thereof (Hepatology Research, 2020; 50: 892-923.), and the like. Specifically, after starting NUC treatment, oral administration of NUC, which was available at that time, was continued.

### (4) Follow-up and liver cancer surveillance

Follow-up and liver cancer surveillance for patients under treatment with NUC administration for HBV were conducted in the same manner as the follow-up and liver cancer surveillance for patients receiving antiviral treatment for HCV.

### (5) Serological test and statistical analysis

Serological tests and statistical analysis of patients under treatment with NUC administration for HBV were performed in the same manner as in those of patients receiving antiviral treatment for HCV.

### B. Results

### (1) In cases of liver cancer development under treatment with NUC administration for HBV, serum GDF15 level was higher than in cases of no liver cancer development.

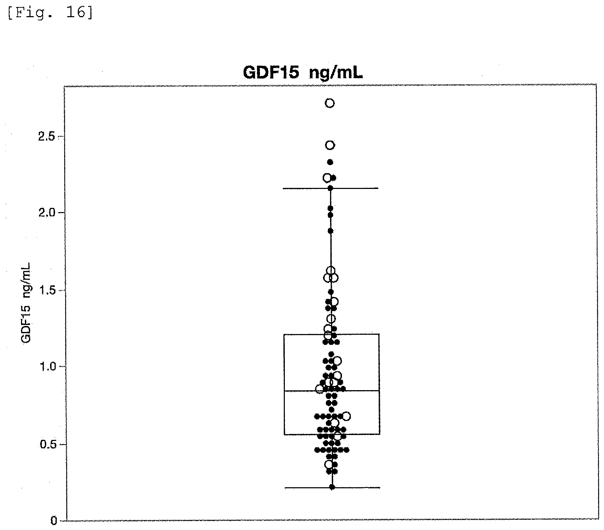

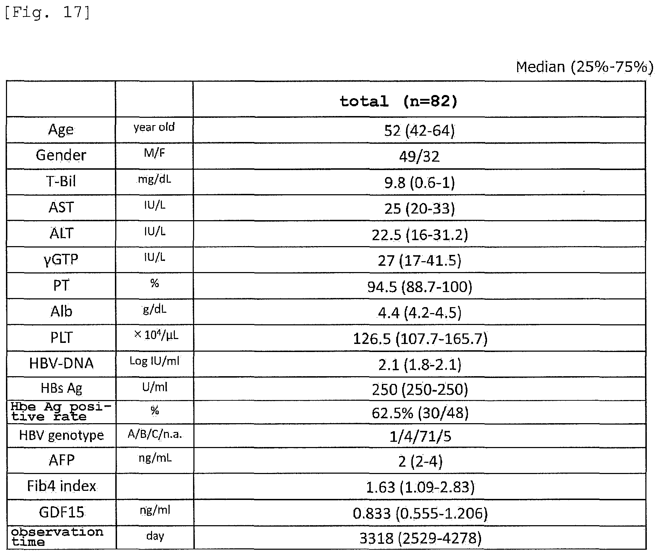

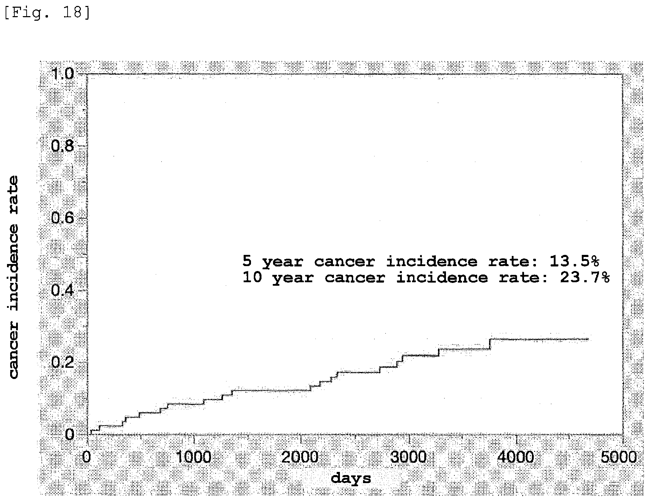

A scatter diagram of serum concentration of GDF15, a graph showing patient background, and a graph showing changes over time in liver cancer incidence rate in the entire cohort at this time in the cases under treatment with NUC administration are respectively shown in Fig. 16, Fig. 17, and Fig. 18. As shown in Fig. 16 and Fig. 17, the median value and 25%-75% interval of GDF serum concentration in the entire cohort were 0.833 ng/mL and 0.555-1.206 ng/mL.

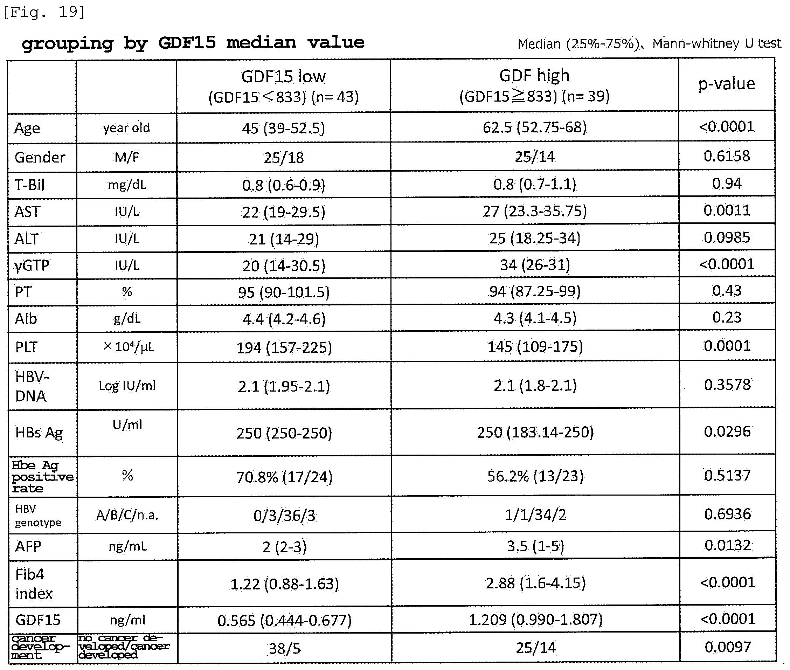

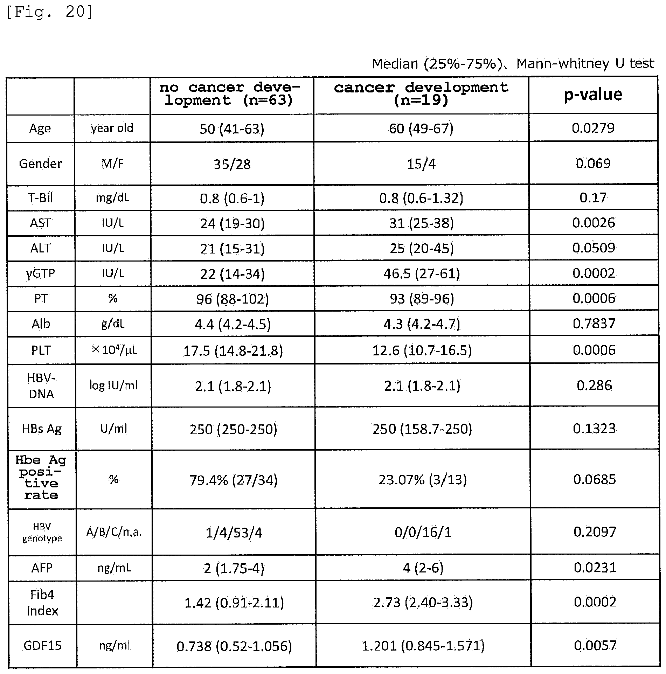

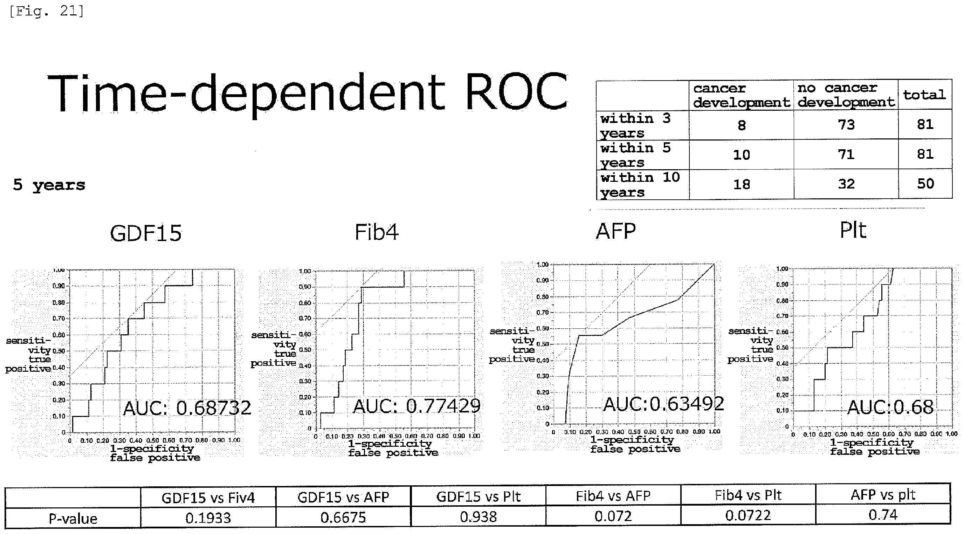

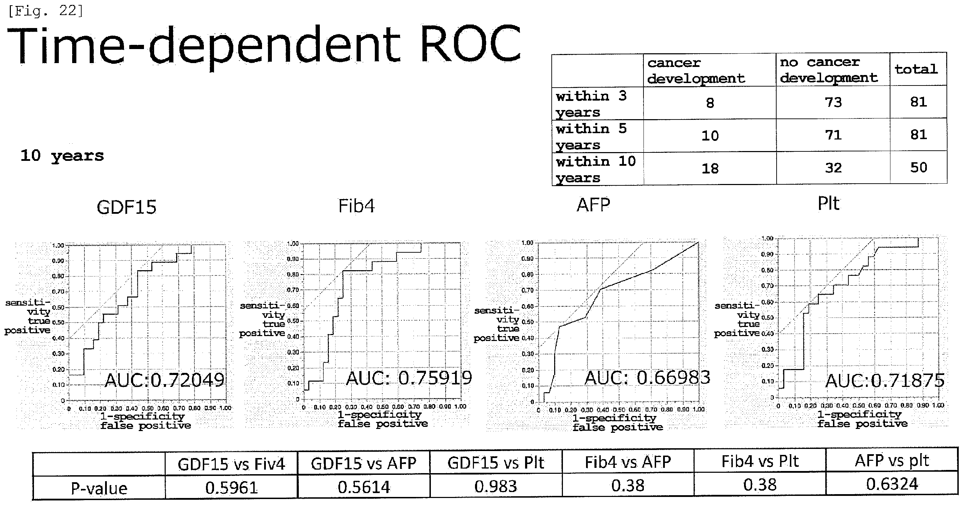

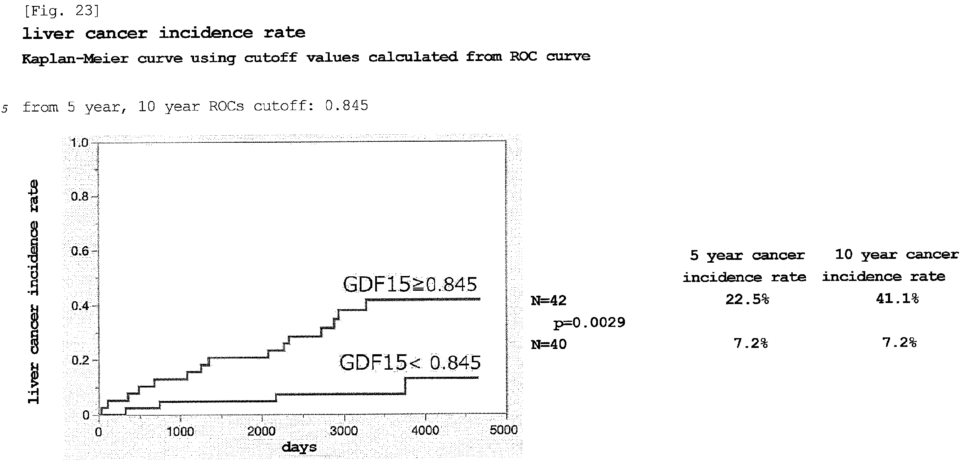

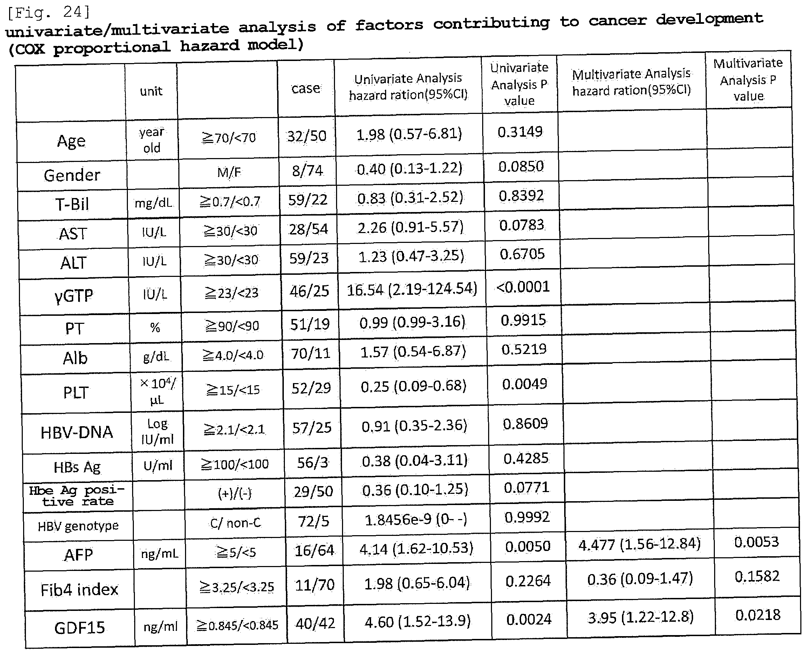

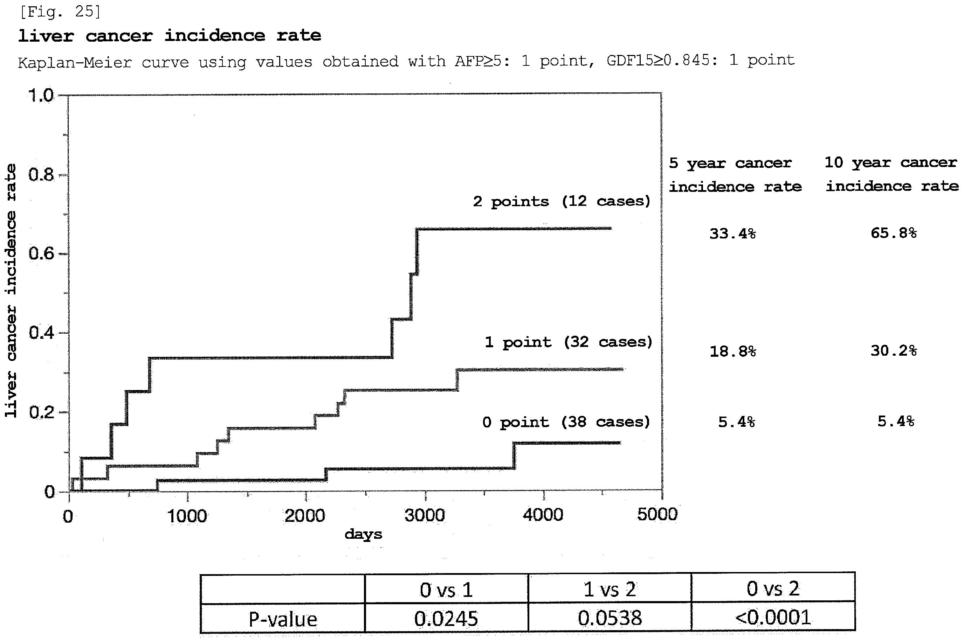

Fig. 19 is a table showing patient backgrounds grouped by median serum concentration of GDF15 (0.833 ng/mL). Fig. 20 is a table showing patient backgrounds grouped by the presence or absence of liver cancer development. Fig. 21 and Fig. 22 are graphs respectively showing the results of time-course ROC (receiver operating characteristic) curve analysis of the presence or absence of cancer development for GDF15, Fib4, AFP, and Plt at 5 and 10 years from the stored serum point. The vertical axis of each graph shows the sensitivity or true positive rate, the horizontal axis shows the false positive rate (1-specificity), and AUC shows the area under the ROC curve of each graph. Fig. 23 is a graph showing changes over time in liver cancer incidence rate using the cutoff value (0.845 ng/mL) determined as the value which makes Youden index the maximum from the ROC curve. Fig. 24 is a table showing the results of univariate/multivariate analysis of factors contributing to cancer development using COX proportional hazards model. Fig. 25 is a graph showing changes over time in liver cancer incidence rate, which was plotted by giving one point to each case with a cutoff value exceeding 5 ng/mL and 0.845 ng/mL respectively for two types of markers of AFP and GDF15 that showed good results in multivariate analysis, and grouping the cases according to the scores. It was shown that stratification by a scoring system that uses the novel biomarker GDF15 alone or in combination with the known marker AFP is useful for predicting liver cancer development in HBV patients under treatment with NUC administration.

From the above results, it was verified that the serum concentration marker of GDF15 is useful for predicting liver carcinogenesis even in subjects who are under treatment with NUC administration for hepatitis B virus (HBV) and have not developed liver cancer.

### Example 3: Evaluation of risk of developing liver cancer in subject affected with NAFL or NASH but has not developed liver cancer

### A. Material and method

### (1) Case population as research target

The research subject of this Example is, among the cases diagnosed with NAFLD by liver biopsy from 2012 to 2020, a case with stored serum during the liver biopsy which permits investigation of progress thereafter. Patients with a history of liver cancer at the time of the serum point and patients with combined liver diseases other than NAFLD are excluded.

### (2) Clinical research review

All the members of the cases participating in the present invention submitted an informed consent document. The design of the present invention complies with the Helsinki Declaration. The patient information and sample collection, analysis protocol in the present invention were approved by the Ethical Review Board of Osaka University Hospital (IRB 17032, 19551), and permitted by each facility including Osaka University Hospital.

### (3) Treatment of NAFL or NASH

Non-alcoholic fatty liver (NAFL) and nonalcoholic steatohepatitis (NASH) were treated in accordance with the guidelines available at that time from NAFLD/NASH SINRRYO GUIDELINE 2020 (NAFLD/NASH clinical practice guideline 2020) (edited by the Japanese Society of Gastroenterology · the Japan Society of Hepatology, revised 2nd edition, published in November, 2020, Nankodo Co., Ltd. (https://www.jsge.or.jp/guideline/guideline/pdf/nafldnash2020.p df)), English version thereof (Tokushige, K. et al. HepatologyResearch.2021; 51:1013-1025. and Tokushige, K. et al. Journal of Gastroenterology 2021; 56: 951-963.) and the like.

### (4) Follow-up and liver cancer surveillance

Follow-up and liver cancer surveillance for patients affected with NAFL or NASH were conducted in the same manner as the follow-up and liver cancer surveillance for patients receiving antiviral treatment for HCV.

### (5) Serological test and statistical analysis

Serological tests and statistical analysis of patients affected with NAFL or NASH were performed in the same manner as in those of patients receiving antiviral treatment for HCV.

### B. Results

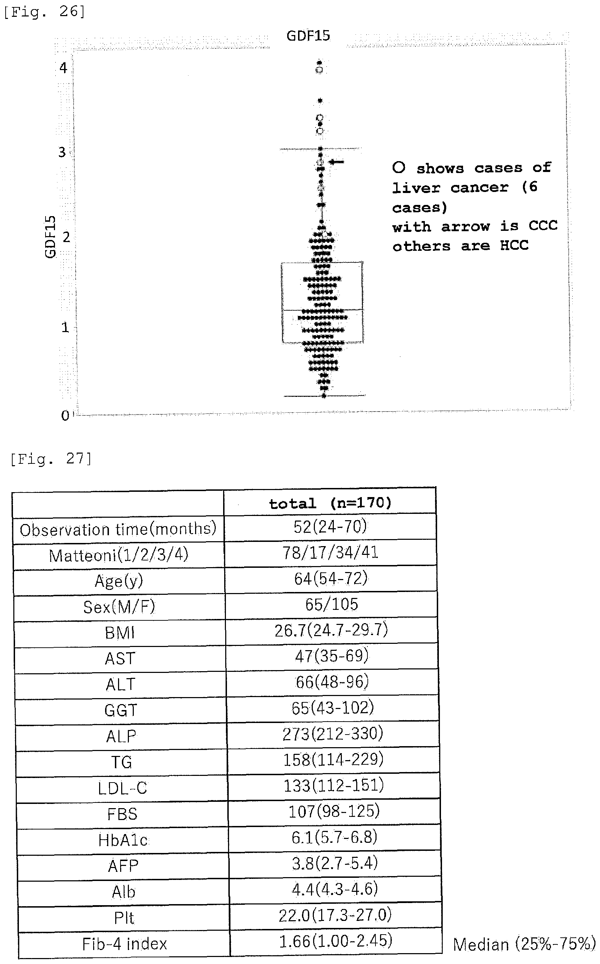

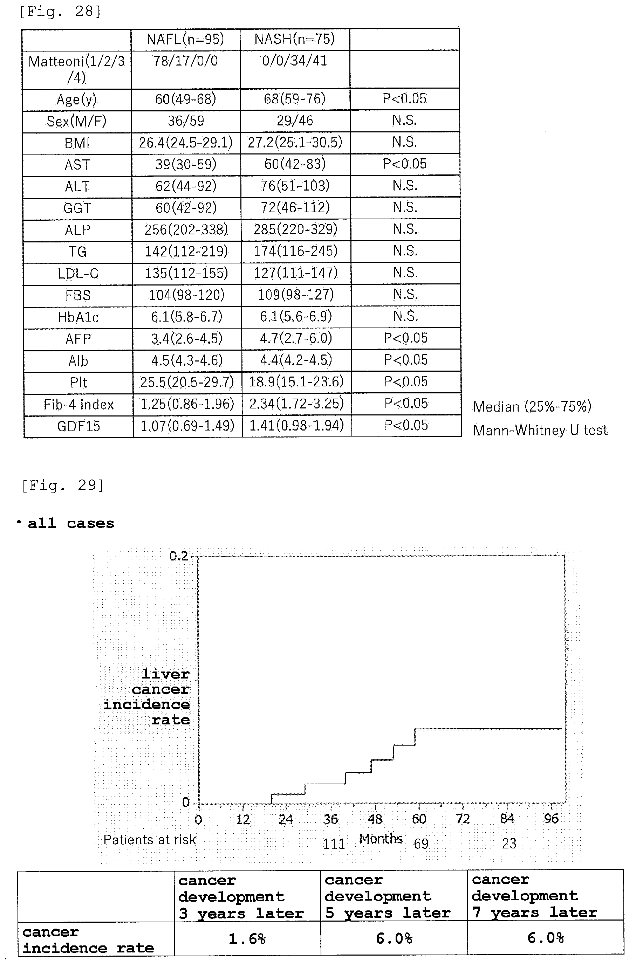

Fig. 26 is a scatter diagram of serum concentrations of GDF15 in patients affected with NAFL or NASH. In the scatter diagram of Fig. 26, black circles are cases without liver cancer development, and white circles are cases with liver cancer development. Five out of six cases of liver cancer development were primary hepatocellular carcinoma (HCC), and one case indicated by an arrow was cholangiocellular carcinoma (CCC). Fig. 27 is a table showing backgrounds of patients affected with NAFL or NASH. Fig. 28 is a table showing patient backgrounds divided into patients affected with NAFL and patients affected with NASH. As shown in Fig. 28, the median value and 25%-75% interval of GDF serum concentration in the entire cohort of patients affected with NAFL were 1.07 ng/mL and 0.69-1.49 ng/mL, and the median value and 25%-75% interval of GDF serum concentration in the entire cohort of patients affected with NASH were 1.41 ng/mL and 0.98-1.94 ng/mL. Fig. 29 is a graph showing changes over time in liver cancer incidence rate in the entire patient cohort affected with NAFL or NASH.

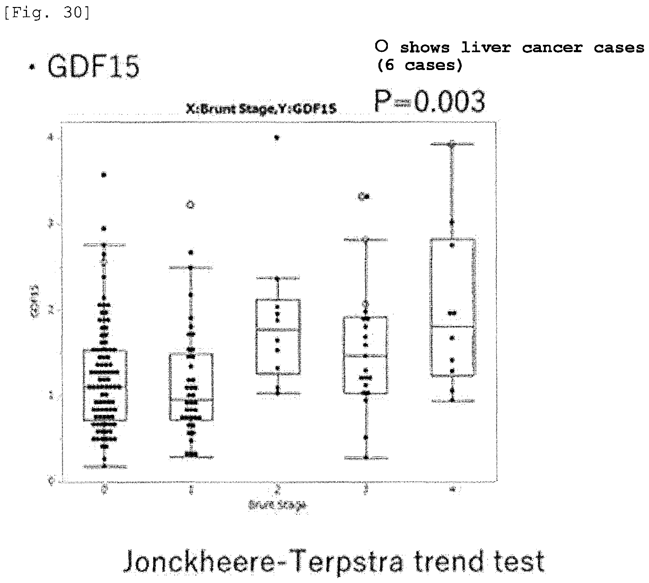

Fig. 30 is a scatter diagram of serum concentrations of GDF15 in cases grouped into Brunt Stage types 0 to 4. The tendency for the serum concentration of GDF15 in each group to increase as the stage progresses from Brunt Stage type 0 to type 4 was verified using the Jonckheere-Terpstra trend test, and the significant difference P was 0.003. Thus, from Fig. 30, a significant correlation was observed between the serum concentration of GDF15 and fibrosis.

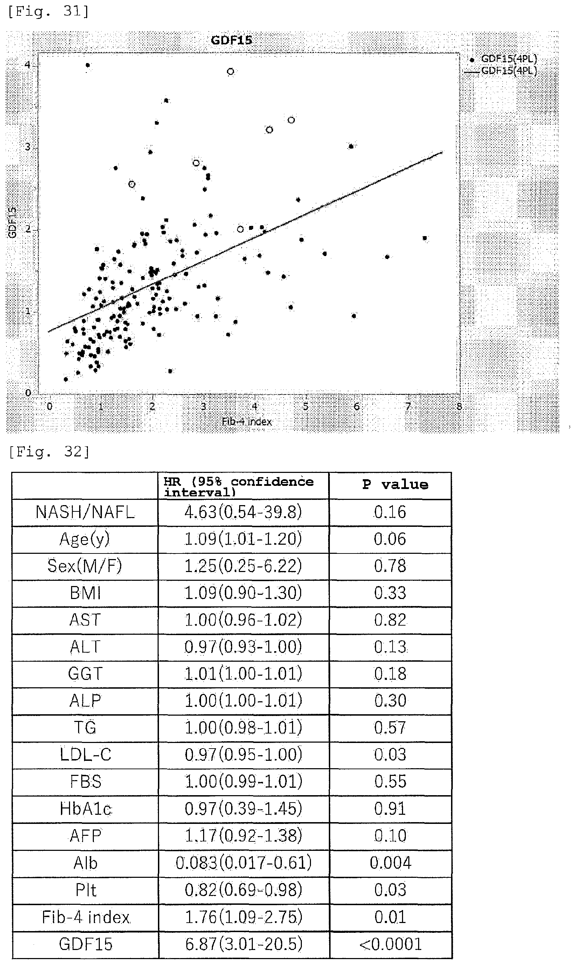

Fig. 31 is a scatter diagram examining the correlation between serum concentration of GDF15 and Fib-4 index in patients affected with NAFL or NASH. The significant difference P was less than 0.0001 and the determination coefficient R² was 0.245, and a significant correlation was observed.

Fig. 32 is a table showing hazard ratios of various attributes, blood markers, Fib-4 index, and the like of patients affected with NAFL or NASH. As is clear from Fig. 32, the P value of GDF15 is less than 0.0001, and strikingly lower than any other attribute, blood marker, Fib-4 index, and the like.

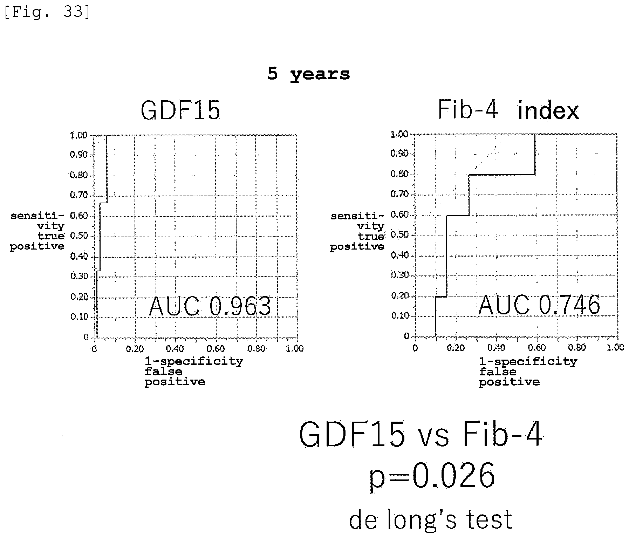

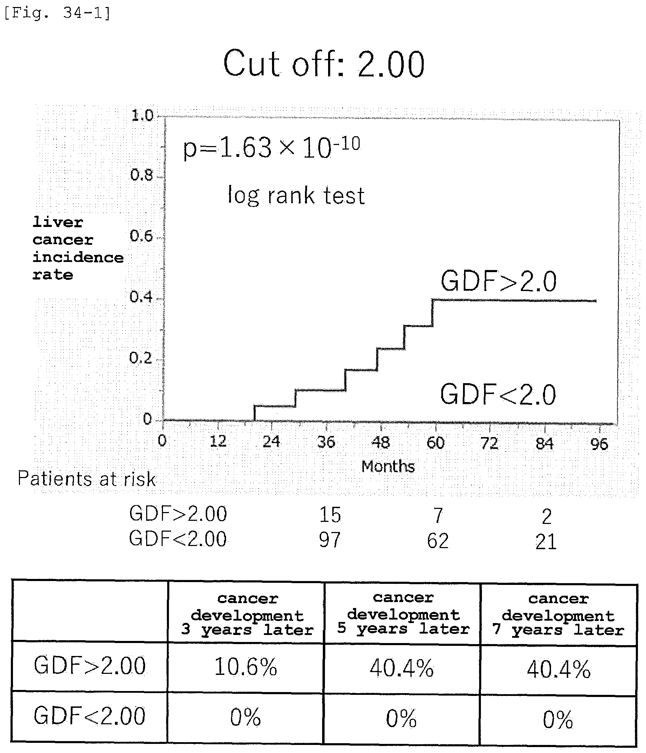

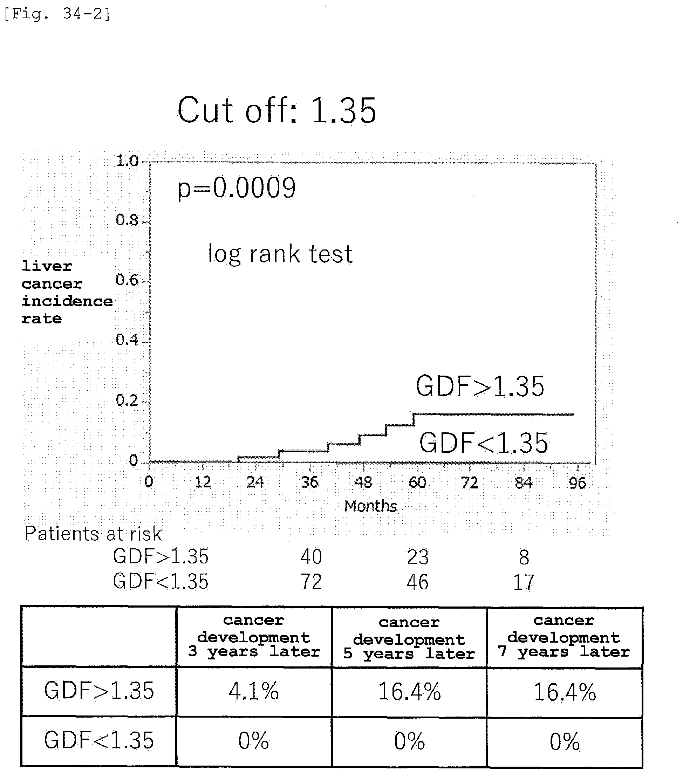

Fig. 33 is a graph showing the results of time-course ROC (receiver operating characteristic) curve analysis of the presence or absence of cancer development for GDF15 and Fib-4 index at 5 years from the stored serum point. The vertical axis of each graph shows the sensitivity or true positive rate, the horizontal axis shows the false positive rate (1-specificity), and AUC shows the area under the ROC curve of each graph. In the cohort of NAFL or NASH patients, liver cancer was developed in 5 cases among the 76 cases subjected to progress observation for 5 years. A comparison of AUC showed that GDF15 has a higher ability to predict the development of liver cancer within 5 years than the Fib-4 index.

34-1 and 34-2 are graphs respectively showing changes over time in liver cancer incidence rate with cutoff values of 2.00 ng/mL and 1.35 ng/mL. 2.00 ng/mL is a cutoff value determined from the ROC curve of cancer development within 5 years in the cohort of NAFL or NASH patients, as the maximum value of Youden index. 1.35 ng/mL is a cutoff value determined from the ROC curve from the derivation cohort of hepatitis C patients who achieved SVR in Example 1, as the maximum value of Youden index. It was shown that the stratification using 2.00 ng/mL as a cutoff value greatly contributes to the prediction of the liver cancer incidence rate of NAFL and NASH.

From the above results, it was verified that the marker of serum GDF15 concentration is useful for predicting liver carcinogenesis even in subjects affected with NAFLD including NAFL and NASH.

### Example 4: Examination with additional cohort at Ogaki Municipal Hospital

An additional 183 cases at Ogaki Municipal Hospital were further examined.

### A. Material and method

### (1) Case population as research target

The research subject of this Example is, among the cases diagnosed with NAFLD by liver biopsy from 2005 to 2020, a case with stored serum during the liver biopsy which permits investigation of progress thereafter. Patients with a history of liver cancer at the time of the serum point and patients with combined liver diseases other than NAFLD are excluded.

### (2) Clinical research review

All the members of the cases participating in the present invention submitted an informed consent document. The design of the present invention complies with the Helsinki Declaration. The patient information and sample collection, analysis protocol in the present invention were approved by the Ethical Review Board of Osaka University Hospital (IRB 17032), and permitted by Osaka University Hospital and Ogaki Municipal Hospital.

### (3) Treatment of NAFLD

The treatment of NAFLD was performed in the same manner as in Example 3.

### (4) Follow-up and liver cancer surveillance

Follow-up and liver cancer surveillance for patients affected with NAFLD were conducted in the same manner as the follow-up and liver cancer surveillance for patients receiving antiviral treatment for HCV.

### (5) Serological test and statistical analysis

Serological tests and statistical analysis of patients affected with NAFLD were performed in the same manner as in those of patients receiving antiviral treatment for HCV.

### B. Results

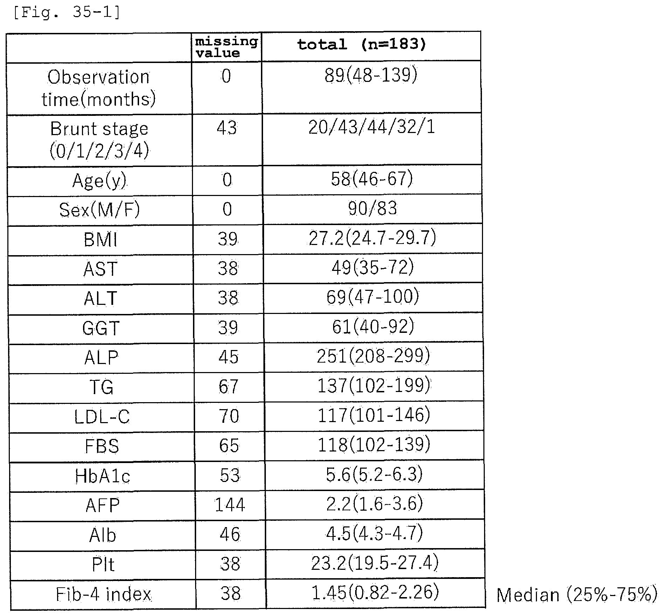

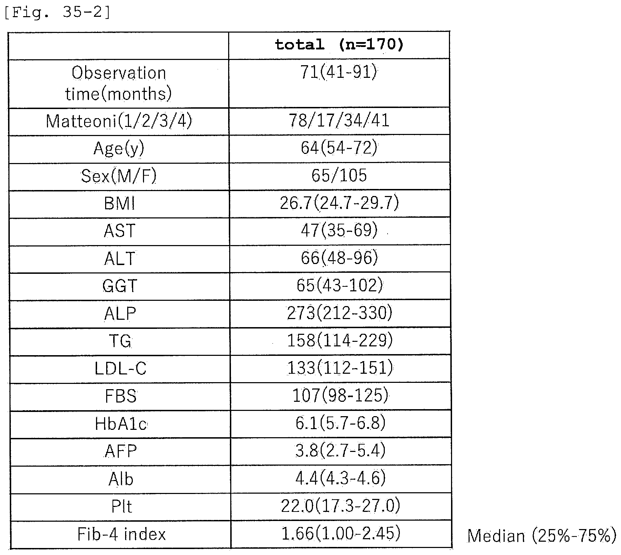

Fig. 35-1 and Fig. 35-2 respectively show patient backgrounds of Ogaki Municipal Hospital and the cohort of Example 3 with an extended observation time.

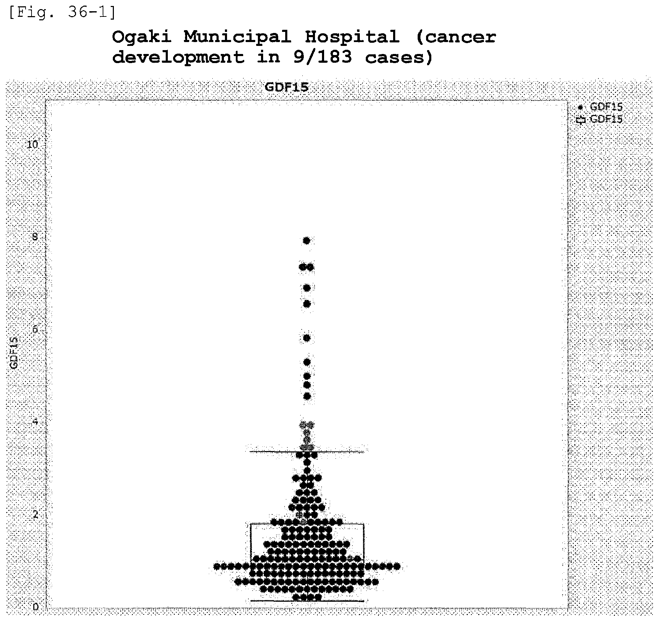

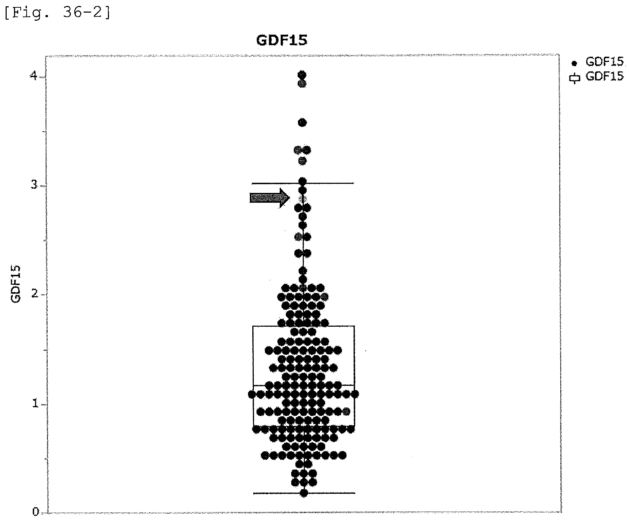

Fig. 36-1 is a scatter diagram of serum concentrations of GDF15 in the patients affected with NAFLD from Ogaki Municipal Hospital. In the scatter diagram of Fig. 36-1, black circles are liver cancer non-development cases, and gray circles are liver cancer development cases. All nine cases of liver cancer development were primary hepatocellular carcinoma (HCC). Fig. 36-2 is a scatter diagram of the serum GDF15 concentration of patients affected with NAFLD in the cohort of Example 3 with an extended observation time. In the scatter diagram of Fig. 36-2, black circles are cases without liver cancer development, and gray circles are cases with liver cancer development. Seven out of eight cases of liver cancer development were primary hepatocellular carcinoma (HCC), and one case indicated by an arrow was cholangiocellular carcinoma (CCC).

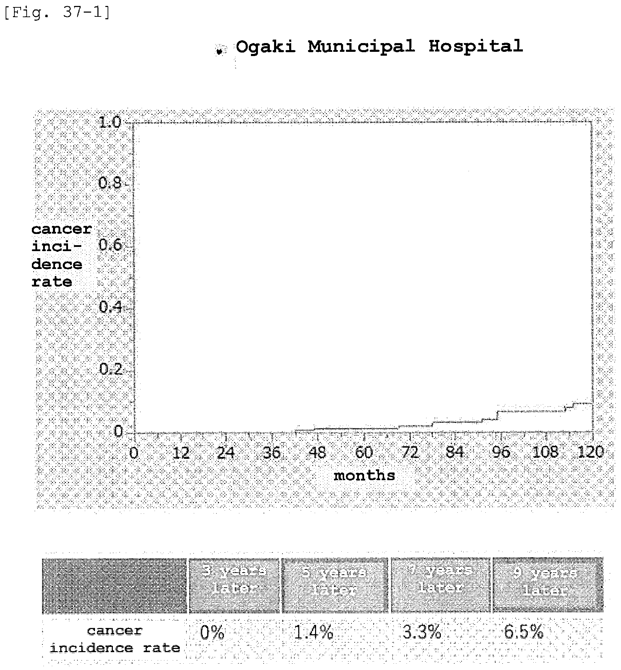

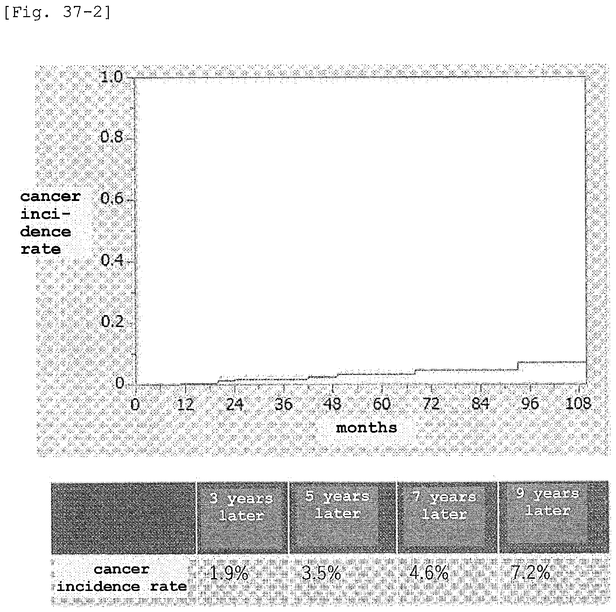

Fig. 37-1 and Fig. 37-2 respectively show changes over the years in liver cancer incidence rate in the Ogaki Municipal Hospital cohort and the cohort of Example 3 with an extended observation time.

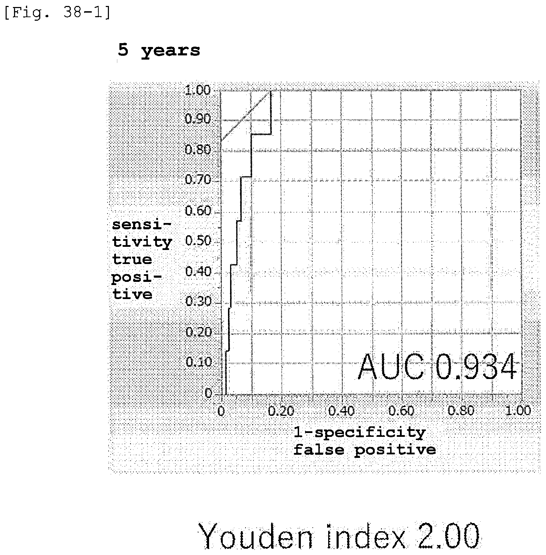

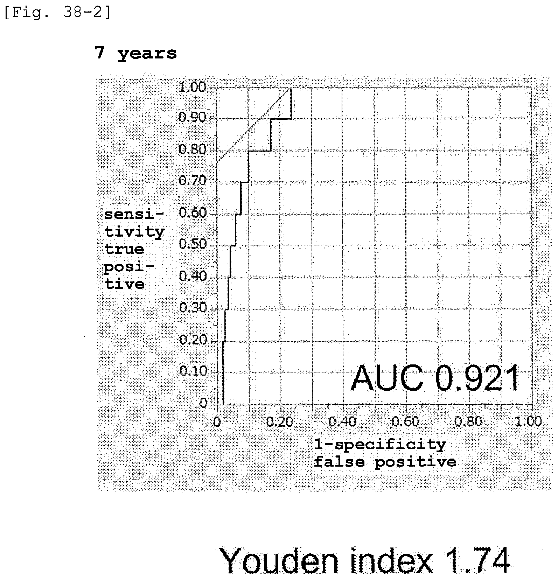

Fig. 38-1, Fig. 38-2, and Fig. 38-3 are graphs showing the results of time-course ROC (receiver operating characteristic) curve analysis of the presence or absence of cancer development at 5 and 7 years from the stored serum point. The vertical axis of each graph shows the sensitivity or true positive rate, the horizontal axis shows the false positive rate (1-specificity), and AUC shows the area under the ROC curve of each graph.

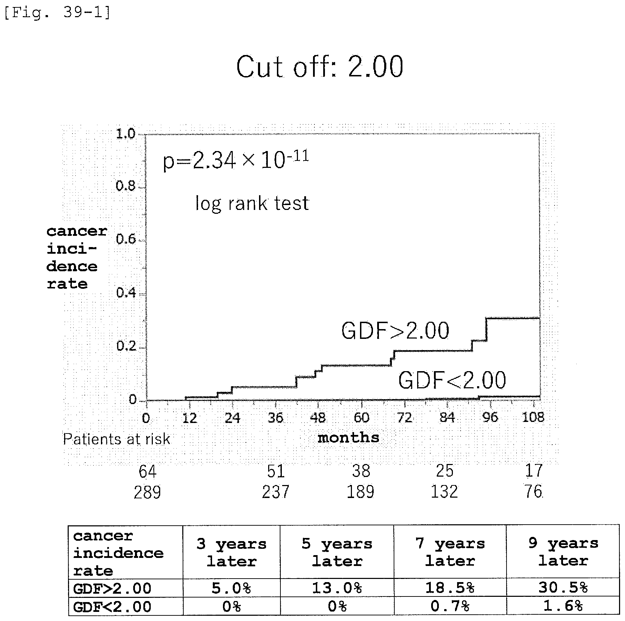

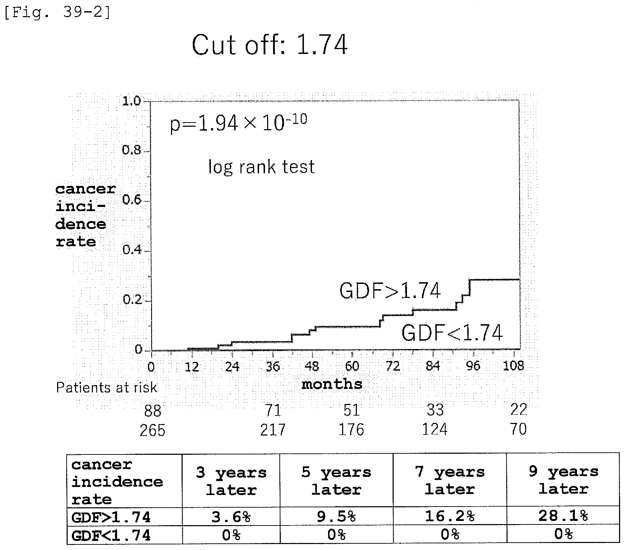

Fig. 39-1 and Fig. 39-2 are graphs respectively showing changes over time in liver cancer incidence rate with cutoff values of 2.00 ng/mL and 1.74 ng/mL for GDF15 for a combination of the Ogaki Municipal Hospital cohort and the cohort of Example 3 with an extended observation time. 2.00 ng/mL is a cutoff value determined from the ROC curve from a cohort of NAFLD patients during the observation time of 5 years, as the maximum value of Youden index. 1.74 ng/mL is a cutoff value determined from the ROC curve from a cohort of NAFLD patients during the observation time of 7 years, as the maximum value of Youden index. It was shown that the stratification using 2.00 ng/mL and 1.74 ng/mL as cutoff values greatly contributes to the prediction of the liver cancer incidence rate of NAFLD.

From the above results, it was verified that the marker of serum GDF15 concentration is useful for predicting liver carcinogenesis also in the examination of the Ogaki Municipal Hospital cohort combined with the cohort of Example 3 with an extended observation time.

This application is based on a patent application No. 2021-121873 filed in Japan (filing date: July 26, 2021) and a patent application No. 2022-084132 filed in Japan (filing date: May 23, 2022), the contents of which are incorporated in full herein by reference.

### [Industrial Applicability]

According to the present invention, it becomes possible to evaluate the risk of developing liver cancer in a subject with higher accuracy based on the GDF15 level. It is also possible to stratify subjects according to the level of the evaluated risk of developing liver cancer and perform gradient distribution so that subjects with a high risk of developing liver cancer can undergo liver cancer screening tests more frequently than subjects with a low risk of developing liver cancer. As a result, both the burden of test on individual subjects and the medical and economic loss for the society as a whole can be reduced.

## Claims

1. A method for evaluating a risk of developing liver cancer in a subject, comprising
(1) a step of measuring a GDF15 level of a subject,
(2) a step of relating the GDF15 level to the risk of developing liver cancer.

2. The method according to claim 1, wherein the subject is at least one type of subject selected from the group consisting of a subject who achieved sustained virological response (SVR) for hepatitis C virus (HCV), a subject under NUC administration for hepatitis B, and a subject with NAFLD.

3. The method according to claim 1 or 2, wherein the GDF15 level of the subject of not less than a cutoff value set in advance is an indicator that the subject has a high risk of developing liver cancer, and the GDF15 level of less than the cutoff value is an indicator that the subject has a low risk of developing liver cancer.

4. The method according to any one of claims 1 to 3, wherein the GDF15 level is the level of GDF15 protein in serum or plasma, and/or the level of GDF15 transcription product in liver tissue or circulating blood.

5. The method according to claim 3, wherein the cutoff value is set based on statistical analysis or ROC analysis of the GDF15 level.

6. The method according to claim 5, wherein the cutoff value is a median value of the GDF15 level of the subject, or a numerical value of the GDF15 level at which a Youden index of an ROC curve of the subject has the maximum value.

7. The method according to claim 6, wherein the subject is a subject who achieved SVR for HCV and the cutoff value of the GDF15 level is a serum GDF15 protein concentration of about 1400 pg/mL, or the subject is a subject under NUC administration for hepatitis B and the cutoff value of the GDF15 level is a serum GDF15 protein concentration of about 845 pg/mL, or the subject is a subject with NAFLD and the cutoff value of the GDF15 level is a serum GDF15 protein concentration of about 2000 pg/mL.

8. The method according to any one of claims 1 to 7, wherein the level of the GDF15 protein in the serum is determined by the ELISA method.

9. The method according to claim 7 or 8, wherein the subject is a subject who achieved SVR for HCV, the serum GDF15 level has a cutoff value of about 1400 pg/mL, and the cutoff values of AFP and FIB-4 indexes are further combined for determination in the step of determining the risk of developing liver cancer.

10. The method according to claim 9, wherein the cutoff values of the AFP and FIB-4 indexes are 5 ng/mL and 3.25, respectively.

11. A kit for use in the method according to any one of claims 1 to 10, comprising an anti-GDF15-specific antibody, and/or a primer pair or probe for specifically detecting the GDF15 transcription product.

12. A diagnostic agent for use in the method according to any one of claims 1 to 10, comprising an anti-GDF15-specific antibody, and/or a primer pair or probe for specifically detecting the GDF15 transcription product.

13. Use of GDF15 as a biomarker for evaluating a risk of developing liver cancer in a subject, comprising
(1) a step of measuring a GDF15 level of a subject, and
(2) a step of relating the GDF15 level to the risk of developing liver cancer.

14. The use according to claim 13, wherein the subject is at least one type of subject selected from the group consisting of a subject who achieved sustained virological response (SVR) for hepatitis C virus (HCV), a subject under NUC administration for hepatitis B, and a subject with NAFLD.
